# EUROPEAN PATENT SPECIFICATION

(11) **EP 1 969 376 B1**
(45) Date of publication and mention of the grant of the patent: **07.07.2010**
(21) Application number: 06830605.9
(22) Date of filing: 13.12.2006
(51) Int. Cl.: G01N 33/68

(54) **AN IN-VITRO METHOD FOR SCREENING ACCESSIBLE BIOLOGICAL MARKERS IN PATHOLOGIC TISSUES**
IN-VITRO-VERFAHREN ZUM SCREENING ZUGÄNGLICHER BIOLOGISCHER MARKER IN KRANKHAFTEN GEWEBEN
PROCÉDÉ DE CRIBLAGE IN VITRO DE MARQUEURS BIOLOGIQUES ACCESSIBLES DANS DES TISSUS PATHOLOGIQUES

(30) Priority: 07.01.2006 EP 06100141
(43) Date of publication of application: 17.09.2008
(60) Divisional application: 10164626.3
(73) Proprietor: Université de Liège, 4031 Angleur (BE)
(72) Inventor: CASTRONOVO, Vincent, B-4000 Liège (BE); WALTREGNY, David, B-4000 Liège (BE); KISCHEL, Philippe, B-4000 Liège (BE)
(74) Representative: Polypatent
(86) International application number: PCT/EP2006/069679
(87) International publication number: WO 2007/080039

(56) References cited:
- WO-A2-01/71359
- US-A1- 2003 073 137
- BUSCH G ET AL: "SELECTIVE ISOLATION OF INDIVIDUAL CELL SURFACE PROTEINS FROM TISSUE CULTURE CELLS BY A CLEAVABLE BIOTIN LABEL" EUROPEAN JOURNAL OF CELL BIOLOGY, WISSENSCHAFLICHE VERLAGSGESELLSCHAFT, STUTTGART,, DE, vol. 50, no. 2, December 1989 (1989-12), pages 257-262, XP001064403 ISSN: 0171-9335
- SCHEURER SIMONE B ET AL: "A comparison of different biotinylation reagents, tryptic digestion procedures, and mass spectrometric techniques for 2-D peptide mapping of membrane proteins." PROTEOMICS AUG 2005, vol. 5, no. 12, August 2005 (2005-08), pages 3035-3039, XP002418329 ISSN: 1615-9853
- FUENTE DE LA E K ET AL: "BIOTINYLATION OF MEMBRANE PROTEINS ACCESSIBLE VIA THE PULMONARY CIRCULATION IN NORMAL AND HPEROXIC RATS" AMERICAN JOURNAL OF PHYSIOLOGY, AMERICAN PHYSIOLOGICAL SOCIETY, BETHESDA, MD, US, vol. 272, no. 3, PART 1, March 1997 (1997-03), pages L461-L470, XP001064381 ISSN: 0002-9513
- RYBAK JASCHA-N ET AL: "IN VIVO PROTEIN BIOTINYLATION FOR IDENTIFICATION OF ORGAN-SPECIFIC ANTIGENS ACCESSIBLE FROM THE VASCULATURE" NATURE METHODS, vol. 2, no. 4, April 2005 (2005-04), pages 291-298, XP008074383 ISSN: 1548-7091
- SCHEURER SIMONE B ET AL: "Identification and relative quantification of membrane proteins by surface biotinylation and two-dimensional peptide mapping." PROTEOMICS JUL 2005, vol. 5, no. 11, July 2005 (2005-07), pages 2718-2728, XP002418330 ISSN: 1615-9853
- RYBAK JASCHA-N ET AL: "Purification of biotinylated proteins on streptavidin resin: a protocol for quantitative elution." PROTEOMICS AUG 2004, vol. 4, no. 8, August 2004 (2004-08), pages 2296-2299, XP002418331 ISSN: 1615-9853
- NARA Y ET AL: "Immunohistochemical localization of extracellular matrix components in human breast tumours with special reference to PG-M/versican" HISTOCHEMICAL JOURNAL, vol. 29, no. 1, 1997, pages 21-30, XP002373603 ISSN: 0018-2214
- RICCIARDELLI ET AL: "Regulation of stromal versican expression by breast cancer cells and importance to relapse-free survival in patients with node-negative primary breast cancer." CLINICAL CANCER RESEARCH, vol. 8, - 2002 pages 1054-1060,
- SUWIWAT ET AL.: "Expression of extracellular matrix components versican, chondroitin sulfate, tenascin, and hyaluronan, and their association with disease outcome in node-negative breast cancer." CLINICAL CANCER RESEARCH, vol. 10, 2004, pages 2491-2498,
- NARA ET AL.: "Immunohistochemical localization of extracellular matrix components in human breast tumours with special reference to PG-M/versican" HISTOCHEMICAL JOURNAL, vol. 29, 1997, pages 21-30,

## Description

The present invention refers to an in-vitro method for screening specific disease biological markers accessible from the extra cellular space in pathologic tissues.

The discovery of biological markers clinically suited for the accurate detection and selective treatment of diseases represents a major medical challenge. Targeting pathologic tissues without affecting their normal counterparts is one of the most promising approaches for improving treatment efficiency and safety (Wu, A. M. & Senter, P. D. Arming antibodies: prospects and challenges for immunoconjugates. Nat Biotechnol 23, 1137-1146 (2005); Adams, G. P. & Weiner, L. M. Monoclonal antibody therapy of cancer. Nat Biotechnol 23, 1147-1157 (2005); Carter, P. Improving the efficacy of antibody-based cancer therapies. Nat Rev Cancer 1, 118-129 (2001)). Indisputably, such advances would represent a breakthrough for the therapy of cancer and other diseases. Hence, the identification of valid biological markers including proteins specifically expressed in diseased tissues, has become of utmost importance (Hortobagyi, G. N. Opportunities and challenges in the development of targeted therapies. Semin Oncol 31, 21-27 (2004); Neri, D. & Bicknell, R. Tumour vascular targeting. Nat Rev Cancer 5, 436-446 (2005)). Yet, even the most recent target discovery strategies based on state-of-the-art, high-throughput technologies have not addressed the limitation, in human tissues, of antigen accessibility to suitable high-affinity ligands such as human monoclonal antibodies bound to bioactive molecules (Adams, G. P. & Weirder, L. M. Monoclonal antibody therapy of cancer. Nat Biotecfinol 23, 1147-1157 (2005); Neri. D. & Bicknell, R. Tumour vascular targeting. Nat Rev Cancer 5, 436-446 (2005).

The identification of biological markers unique to specific pathologic processes would be most valuable for the accurate detection (e.g. by imaging studies) and selective therapy of diseases including cancer. For instance, patients suffering from cancer would certainly benefit from such developments. Indeed, chemotherapeutic agents, usually designed to take advantage of tumor cell characteristics such as high proliferation rates, unfortunately also target normal cycling cells including hematopoietic progenitors. The targeted delivery of these drugs and other bioactive molecules (e.g. radioisotopes, cytokines) to the tumor microenvironment (e.g. proteins specifically expressed in the stromal or vascular compartment of the tumor) by means of binding molecules such as recombinant human antibodies, would represent a considerable therapeutic improvement. This selective strategy would increase the amount of drugs reaching the tumor with little or no toxicity to the host's healthy tissues. The recent development of high-throughput proteomic technologies such as mass spectrometry have facilitated the rapid and accurate identification of small, but complex biological sample mixtures, overcoming many limitations of two-dimensional gel electrophoresis for proteome analysis (Peng, J. & Gygi, S. P. Proteomics: the move to mixtures. J Mass Spectrom 36, 1083-91 (2001)) and making target identification easier than ever. The recent development of this high-throughput proteomic technology holds great promise for speeding up the discovery of novel targets, notably in cancer research. For example, gel-free shotgun tandem mass spectrometry has been recently used to compare global protein expression profiles in human mammary epithelial normal and cancer cell lines (Sandhu, C. et al., Global protein shotgun expression profiling of proliferating mcf-7 breast cancer cells. J Proteome Res 4, 674-89 (2005)). Unfortunately, a significant pitfall associated with such an approach is that it provides no clue as to whether proteins of interest are accessible to suitable high-affinity ligands, such as systemically delivered monoclonal antibodies, in human tissues. Indeed, specific, yet poorly accessible proteins expressed in pathologic tissues are expected to be of little value for the development of antibody-based anti-cancer therapies. Strategies that would unveil disease biological markers not only specifically expressed in pathologic tissues but also accessible from the extracellular fluid would help overcome this limitation.

Further, methods are known allowing protein labelling either by *in vivo* terminal perfusion or by *ex vivo* perfusion of human pathologic organs. However, the perfusion technique is restricted to experimental animals (e.g. rodents) or surgically resected organs vascularized by a catheterizable artery (e.g. kidney), which excludes many types of pathologic tissues from investigation, namely those which can not be perfused.

The document Busch et al. (European Journal of Cell Biology, vol. 50, no. 2, pages 257-262 (1989)) refers to the selective isolation of cell surface proteins from tissue culture by a cleavable biotin label. As described isolated erythrocytes or hepatocytes were incubated with a labeled biotin.

The document Scheurer et al. (Proteomics 2005, vol. 5, no. 12, pages 3035-3039) discloses a similar method for labelling cell surface protein using a biotinylation reagent. As described, cells were harvested and used for labelling.

The document De La Fuente et al. (American Journal of Physiology, vol. 272, no: 3, part 1, pages L461-L470 (1997)) refers to the labelling of membrane proteins. Either perfusion of an animal or labelling of a homogenate is used for this method.

The document Rybak et al. (Nature Methods, vol. 2, no.4, 291-298 (2005)) describes a method for the biotinylation of identification of organ-specific antigens which are accessible from the vasculature. The method described makes use of a "terminal perfusion" step of an animal. In an alternative method described in this document an *ex vivo* perfusion procedure of organs is used.

The document WO 01/71359 A2 refers to methods to label and isolate lumen-exposed polypeptides that are expressed on the luminal side of cells lining, vascular, ductal and other perfusible tissue spaces. The method makes use of a step of administering a labelled reagent into the perfusible space in an intact organ or an intact animal. As described herein the methods comprise administering the reagent into the perfusible space of the intact organ or tissue or the intact animal by administration of the reagent; in particular the described methods make use of perfusion of the sample.

The object of the present invention therefore was to provide a new, simple, quick and efficient method to identify, in human tissues originating from biopsies and non-perfusable organs (e.g. cancer lesions present in mastectomy or radical prostatectomy specimens), specific disease biological markers accessible from the extracellular space.

The object is solved by an in vitro method for screening specific disease biological markers which are accessible from the extra cellular space in pathologic tissues for high-affinity ligands comprising the steps of:
- immersion of a native normal tissue sample in a solution containing a labelling reagent for labelling proteins; wherein accessible proteins are labelled by the labelling reagent;
- immersion of a native pathologic tissue sample in a solution containing a labelling reagent for labelling proteins, wherein accessible proteins are labelled by the labelling reagent;
- separate purification of the labelled proteins of each of the samples;
- analysis of the labelled proteins or fragments thereof of normal tissue and pathologic tissue, respectively;
- determination of the differential expression pattern of the labelled proteins in the native pathologic tissue samples compared to the normal tissue samples;
- judging that the labelled protein(s) having higher expression in the native pathologic tissue sample compared to the normal tissue sample or being expressed more frequently in respective native pathologic tissue samples compared to the normal tissue sample is/are biological marker(s) for pathologic tissue, which are accessible for high-affinity ligands from the extra cellular space.

It is understood that the in the step of immersing a native pathologic tissue sample in a solution containing a labelling reagent for labelling proteins, only accessible proteins are labelled by the labelling reagent; while non-accessible proteins are not or essentially not labelled. In a preferred embodiment of the method it is judged that said labelled protein(s) is/are accessible for high-affinity ligands from the extra cellular space in the native tissue, most preferred is/are accessible for high-affinity ligands from the extra cellular space *in vivo.*

The present invention therefore provides a new chemical proteomic method for the reliable identification of target proteins from diseased tissues, which *in vivo* are accessible from the extra cellular fluid and thus from the bloodstream. By applying this procedure, for example, to small, surgically obtained samples of normal and cancerous human breast tissues, a series of accessible proteins selectively expressed in breast cancer are identified. Some of these breast cancer-associated antigens correspond to extracellular proteins including extra cellular matrix and secreted proteins, and appear to be interesting targets for antibody-based anti-cancer treatments. This powerful technology can theoretically be applied to virtually any tissue and pathologic condition and may become the basis of custom-made target therapies.

According to the present invention the pathologic tissue sample and the normal tissue sample are native until the immersion step. As used herein the term "native tissue sample" means that the tissue sample is not denatured and not fixed. As used herein the term "native tissue" comprises native tissue samples as well as the corresponding tissues *in vivo*.

According to the present invention biological markers for pathologic diseases which are not accessible from the extracellular space in a native tissue sample (and also are not accessible from the extracellular space *in vivo*) will not or will essentially not be labelled.

An important feature of the present invention is that the native tissue samples are immersed in a solution comprising a reactive compound which is marking and labelling proteins which are accessible from the extra cellular space, by simple diffusion through the native tissue. This means that once the tissue sample is immersed into the solution the reactive compound comprised therein will diffuse through the extracellular space of the native tissue sample and thereby brought into contact with accessible proteins and react with them. The labelled proteins can be easily purified and analysed and identified by proteomic methods. Identification of the labelled proteins is, for example, achieved by liquid chromatography and subsequent mass spectrometry. One major advantage is that the present method does not depend on the possibility that the (native) tissue samples used can be perfused or not. Therefore, according to the method of the present invention any tissue can be investigated and explored in order to seek for accessible biological markers.

As used herein the term "biological marker" represents proteins or polypeptides (which may be modified for example by glycosylation), which are expressed in a given pathologic tissue and which are essentially not expressed in normal tissues, or are expressed on a higher level in the given pathologic tissue than in normal tissues, wherein the biological marker indicates a pathologic condition compared to the normal physiologic condition of normal tissues.

As used herein the term "normal tissue" represents either normal tissue corresponding to the pathologic tissue from the same individual or normal tissue corresponding to the pathologic tissue from other individuals or normal tissue that is not related (in extenso either from a different location in the body, or with a different histologic type) to the pathologic tissue either from the same individual or form other individuals.

In a preferred embodiment the term "normal tissue" refers to the normal tissue corresponding the pathologic tissue from the same individual.

According to the present invention the step of determination of the "differential expression pattern" of the labelled proteins in, pathologic tissue samples compared to normal tissue samples means that it is examined, for example, if a given labelled protein is at all expressed in a tissue or not, either pathologic or normal. This analysis may also comprise the assessment of the relation of expression level in pathologic tissue versus normal tissue. Further, this analysis may also comprise the assessment of in how many samples (eventually from different individuals) of a given type of pathologic tissue expression of a given protein is fond compared to in how many samples (eventually from different indivuduals) of the given type of the corresponding or unrelated normal tissue expression of a given protein is found.

According to the present invention there is a step of judging that the labelled protein(s) having higher expression in the native pathologic tissue samples compared to normal tissue samples or being expressed more frequently in respective native pathologic tissue samples compared to normal tissue samples is/are biological marker(s) for pathologic tissue, which are accessible for high-affintiy ligands from the extra cellular space in the native tissue. Most preferred, it is judged that the labelled protein(s) having expression in the native pathologic tissue sample but which is not or essentially not expressed in the corresponding and/or unrelated normal tissue is/are biological marker(s) for pathologic tissue, which are accessible for high-affintiy ligands from the extra cellular space in the native tissue.

Further preferred the labelling reagent for labelling proteins is a reactive biotin, preferably a biotin reactive ester derivative.

In yet another preferred embodiment the purification step makes use of the label of the labelled proteins as selective marker. It is preferred that the labelled proteins are purified by affinity purification mediated by the label.

Preferably, the label is a biotin residue and purification is performed using streptavidin bound to a resin, wherein the biotin-labelled proteins are bound to the resin via streptavidin.

According to a further preferred embodiment after the purification step the labelled proteins are cleaved to peptides, preferably by proteolytic digestion.

Further preferred, the analysis step comprises mass spectrometry, preferably microsequencing by tandem mass spectrometry.

Preferably, the native pathologic tissue sample is derived from tissues selected from the group consisting of tumor tissue, inflamed tissue, atheromatotic tissue or resulting from degenerative, metabolic and genetic diseases.

Further preferred, accessibility of the biological markers refers to being accessible for high-affinity ligands from the extra cellular space. This means that substances (high-affinity ligands) can diffuse *in vitro* through the extra cellular space of a given native tissue to accessible biological markers (which are accessible from the extra cellualr space in the native tissue, and therefore also *in vivo*). In consequence, if a specific substance for labelling is able to diffuse *in vitro* through the extra cellular space of a given pathologic tissue and reaches the biological marker, which is indicating the pathologic condition, then said biological marker (which can be determined according to the present invention) likely will also be accessible *in vivo* via the extracellular space (extra cellular liquid) and therefore can be considered to be a candidate target protein (biological marker) for detecting the disease (for detecting the presence (diagnosis), and/or evaluating the spread (staging) and/or assessing the evolution (monitoring) of the disease) and for targeting drugs to the cells and tissues expressing said accessible protein (biological marker). Preferably, the substances (high-affinity ligands) which can diffuse *in vitro* through the extra cellular space of a given tissue to accessible biological markers are selected from the group consisting of antibodies, antibody fragments, drugs, prodrugs, ligands, biotin, and derivatives and conjugates thereof, preferably conjugates of antibodies or antibody fragments with drugs or prodrugs.

In a further preferred embodiment the biological markers are proteins or polypeptides, which are expressed in the given pathologic tissue and not expressed in the corresponding normal tissue, or are expressed on a higher level in the given pathologic tissue than in the corresponding normal tissue, wherein the biological marker indicates a pathologic condition compared to the normal physiologic condition of the corresponding normal tissue.

In the present patent application a new, simple, quick and efficient method is provided to identify, in human tissue biopsies, specific disease biological markers accessible from the extra cellular space. The method of the present invention preferably makes use of covalent linking of biotin onto primary amines of accessible proteins brought into contact with a solution of reactive biotin ester derivatives. The method according to the present invention permits the *ex vivo* biotinylation of human tissues, which originate either from biopsies or from non-perfusable organs (e.g. cancer lesions present in mastectomy or prostatectomy samples), by simple immersion in the biotinylation solution, without prior lysis or homogenisation, and preferably also without denaturation or fixation. Biotinylated proteins are easily purified thanks to the extremely high and specific interaction of biotin with streptavidin, even in lysis buffers containing strong detergents, thus minimizing the non-specific binding during the affinity purification step. Proteolytic digestion of the purified biotinylated proteins preferably is performed directly on-resin, before shotgun mass spectrometry analysis.

In summary, the method of the present invention allows the identification of several accessible proteins differentially expressed in pathologic and healthy tissue samples. This method is applicable to virtually any tissue, including tumor tissue samples as well as other pathologic tissues resulting from inflammatory, degenerative, metabolic, and even genetic diseases. The present invention sought for specific biological markers of human breast cancer, the most frequent form of cancer and the second leading cause of cancer death in American women. Among a list of candidate markers, two of them, versican and periostin, were identified in breast cancers with the chemical proteomic-based approach of the present invention, and were further validated by immunohistochemistry. The accessibility of these extra cellular matrix proteins may be particularly suited for targeting strategies using an intravenous route. It was demonstrated that the method is easy to perform and reliable. Of note, results can be obtained in less than one week. The method is versatile enough to be exploited in the future for complete mapping of primary tumors and associated metastases. It would enable the development of custom-made treatments, targeting only accessible proteins effectively expressed in the diseased tissues. It may be anticipated that once the most relevant, accessible biological markers specific for a patient's disease are identified, high affinity ligands, such as recombinant human antibodies and their fragments, can be prepared to assess the precise localization of the pathologic lesions and to selectively destroy them. As an example, the human antibody L19, a specific ligand of the EDB domain of fibronectin, a biological marker of several cancer types (Zardi, L. et al. Transformed human cells produce a new fibronectin isoform by preferential alternative splicing of a previously unobserved exon. Embo J 6, 2337-2342 (1987); Pini, A. et al. Design and use of a phage display library. Human antibodies with subnanomolar affinity against a marker of angiogenesis eluted from a two-dimensional gel. J Biol Chem 273, 21769-21776 (1998); Castellani, P. et al. Differentiation between high- and low-grade astrocytoma using a recombinant antibody to the extra domain-B of fibronectin. Am J Pathol 161, 1695-1700 (2002)), is currently tested in clinical trials, both as a imaging tool (conjugated to radioactive iodine (Berndorff, D. et al. Radioimmunotherapy of solid tumors by targeting extra domain B fibronectin: identification of the best-suited radioimmunoconjugate. Clin Cancer Res 11, 7053s-7063s (2005); Borsi, L. et al. Selective targeting of tumoral vasuclature: comparison of different formats of an antibody (L19- to the ED-B domain of fibronectin. Int J Cancer 102, 75-85 (2002)) and as a therapeutic agent (fused with human interleukin-2 (Ebbinghaus, C. et al. Engineered vascular-targeting antibody-interferon-gamma fusion protein for cancer therapy. Int J Cancer 116, 304-313 (2005); Menrad, A. & Menssen, H. D. ED-B fibronectin as a target for antibody-based cancer treatments. Expert Opin Their Targets 9, 491-500 (2005); Carnemolla, B. et al. Enhancement of the antitumor properties of interleukin-2 by its targeted delivery to the tumor blood vessel extracellular matrix. Blood 99, 1659-1665 (2002))). The new technology of the present invention will promote the development of selective target therapies and therefore may represent an unprecedent step toward a clean and effective war against diseases and particularly cancer.

**Best Mode for carrying out the present invention**

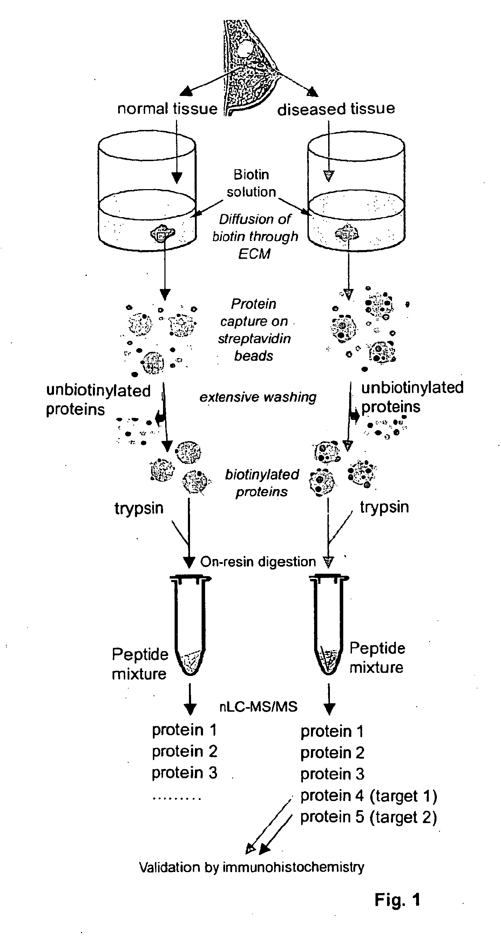

In a preferred embodiment the object is solved by an *in vitro* method for screening specific disease biological markers which are accessible from the extra cellular space in pathologic tissues for high-affinity ligands comprising the steps of:
- immersion of a native normal tissue sample in a solution containing a reactive biotin for labelling proteins; wherein accessible proteins are labelled (biotinylized) by the reactive biotin;
- immersion of a native pathologic tissue sample in a solution containing a reactive biotin for labelling proteins; wherein accessible proteins are labelled (biotinylized) by the reactive biotin;
- separate purification of the biotinylized proteins of each of the samples using a streptavidin-bound resin, wherein the streptavidin moiety binds to the biotin group;
- analysis of the labelled proteins or fragments thereof of normal tissue and pathologic tissue, respectively, using liquid chromatography followed by mass spectrometry;
- determination of the differential expression pattern of the labelled proteins in the native pathologic tissue samples compared to the native normal tissue samples;
- judging that the biotinylized protein(s) having expression in the native pathologic tissue sample but which is/are not or essentially not expressed in the corresponding normal tissue is/are biological marker(s) for the given pathologic tissue, which are accessible for high-affintiy ligands from the extra cellular space in the native tissue. Figure 1 summarizes these steps of the method.

**Examples**

**1. Assessment of the immersion time of the tissue in the biotin solution**

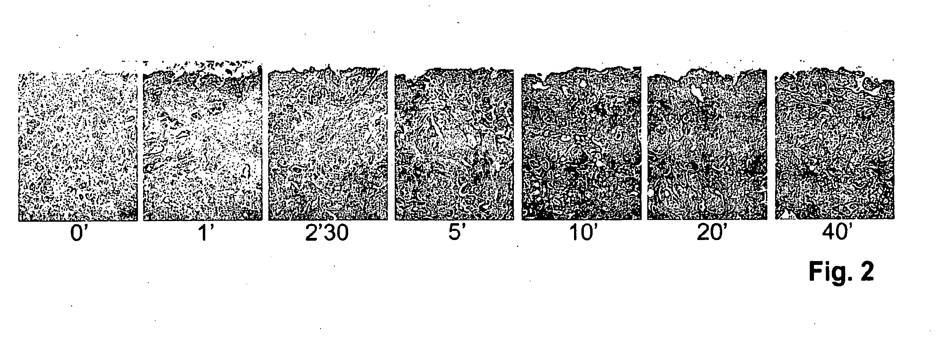

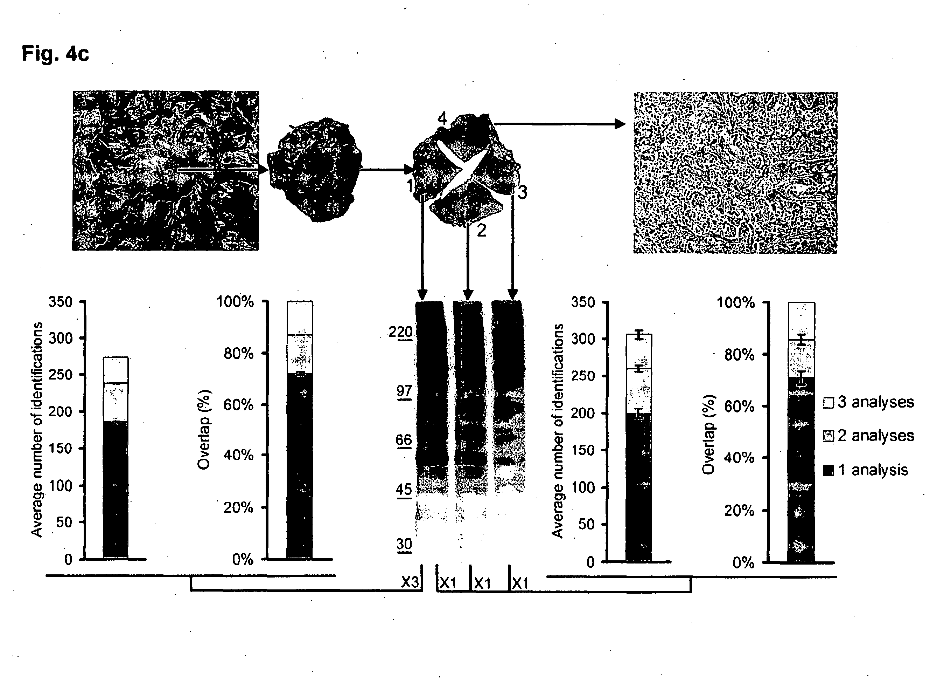

First, the effect of immersion time in the biotin solution on labelling extent and intensity was investigated by histochemistry. Diffusion extent was dependent not only on soaking time, but also on the thickness and composition of the tissue. Increased soaking times (up to 40 minutes) were tested onto breast tissue specimens of variable thicknesses. It was observed that labelling extent, indicative of biotin penetration in the tissues, increased over time (Fig. 2). Since a 20-minute immersion time was found appropriate for 2mm-thick human breast tissue slices, this procedure was used in all further experiments. Reproducibility of the biotinylation step, evaluated by examining labelled protein patterns of different samples from the same tumor specimen, was found consistent (Fig. 4c).

**2. Expression profiles of accessible proteins in breast carcinomas**

Expression profiles of biotinylated, accessible proteins in 7 ductal and 3 lobular human breast carcinomas (Table 2) and their matched non-tumoral counterparts were then determined using the MudPIT (Multidimensional protein identification technology) technique, based on 2-dimensional separation of tryptic peptide digest using nanoflow liquid chromatography coupled to electrospray tandem mass spectrometry. The complete list of proteins includes 670 proteins identified with high confidence (Table 3). Reproducibility of the method was assessed by comparing the protein lists generated by multiple runs of the same sample, and by comparing protein lists generated from 3 different samples from the same tumor (Fig. 4c). The number of proteins identified in the biotinylated breast samples (>250 proteins) was definitely higher than the one found in non-biotinylated, control samples (no more than 10 proteins in normal or tumor samples, data not shown). Proteins found in non-biotinylated samples were most likely "sticky" proteins that unspecifically bound to the str-eptavidin beads and consisted mainly of serum proteins (e.g. human serum albumin, hemoglobin chains and immunoglobulins) and keratins. Biotinylated proteins included extra cellular, plasma membrane, and intracellular proteins (Table 3). As already described in the prior art, i) intracellular proteins are most likely released by damaged cells prior to biotinylation when tissues are sliced, ii) cytoplasmic proteins could be co-purified because of a strong interaction with membrane proteins, and iii) although negatively charged, the long chain biotin esters can enter into the cells (Scheurer, S. B. et al., Identification and relative quantification of membrane proteins by surface biotinylation and two-dimensional peptide mapping. Proteomics 5, 2718-28 (2005); Peirce, M. J. et al., Two-stage affinity purification for inducibly phosphorylated membrane proteins. Proteomics 5, 2417-21 (2005)).

The comparative analysis of non-tumoral and cancerous breast tissues identified several proteins known to be preferential localized in the extra cellular compartment. Stromal proteins that are selectively expressed in cancer tissues are prime candidates for tumor targeting strategies because (i) they are expected to be more accessible than intracellular proteins, (ii) they are often present in high quantities, and (iii) tumor cells frequently induce changes in the stromal compartment. This "reactive stroma" creates a permissive and supportive environment contributing to cancer progression (Walker, R. A. The complexities of breast cancer desmoplasia. Breast Cancer Res 3, 143-145 (2001); De Weaver, O. & Mareel, M. Role of tissue stromal in cancer cell invasion. J Pathol 200, 429-447 (2003)). For these reasons, identification of new stromal proteins specifically involved in cancer is of high interest. Searching for accessible extra cellular matrix (ECM) proteins, versican has been identified as being systematically expressed only in the breast cancer microenvironment (Table 1). Among other potential extra cellular biological markers, periostin and fibronectin were more frequently detected in the cancerous tissues analyzed. Further, the *in situ* expression of some of these potential markers was investigated by immunohistochemistry in a series of human breast cancers together with their normal counterparts.

Table 1 shows a selection of 10 accessible proteins identified with high confidence. Several proteins of this list appeared to be cancer- or breast cancer-associated proteins (e.g. cytokeratins, anterior gradient protein homolog 2, periostin and versican, among others). Interestingly, the membrane antigen ErbB2 was also identified by MS in the single tumor (out of the 10 tumors tested) that was considered as ErbB2-positive by routine immunohistochemical assessment. Biotinylated proteins included extracellular and plasma membrane proteins, but also included a non-negligible fraction of intracellular proteins. The reasons for this observation include intracellular protein leakage when tissues are sliced, biotin penetration, and strong interactions between cytoplasmic and membrane proteins. Nevertheless, the comparative analysis of non-tumoral versus cancerous breast tissues identified several proteins known to be preferentially localized in the extracellular compartment.

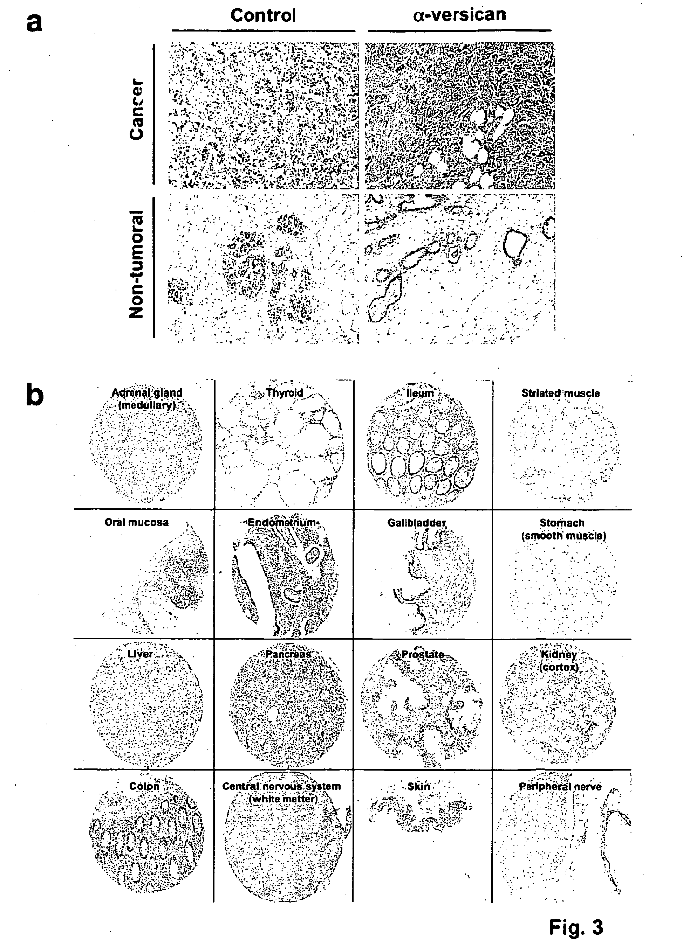

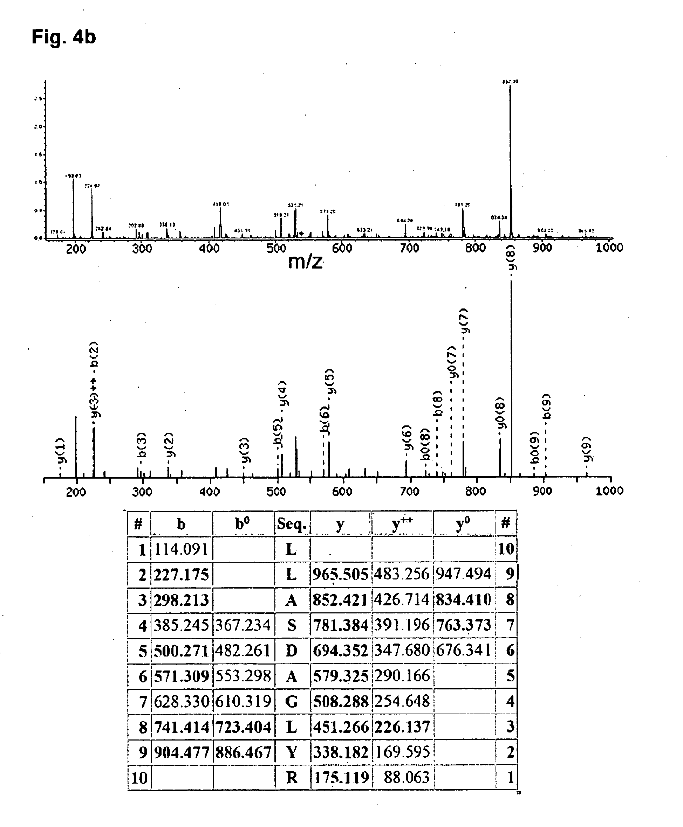

Searching for accessible extracellular matrix (ECM) proteins, versican was identified in this study as being systematically and specifically (10 out of the 10 sample pairs tested) detected in the breast cancer samples, but not in the matched normal counterparts (Table 1). Versican, a large proteoglycan secreted by stromal cells in the ECM, is a recognized cell adhesion and motility modulator that may facilitate tumor cell invasion and metastasis (Yamagata, M., et al., Regulation of cell-substrate adhesion by proteoglycans immobilized on extra cellular substrates. J Biol Chem 264, 8012-8 (1989); Evanko, S. P. et al., Formation of hyaluronan- and versican-rich pericellular matrix is required for proliferation and migration of vascular smooth muscle cells. Arterioscler Thromb Vasc Biol 19, 1004-13 (1999); Ricciardelli, C. et al., Regulation of stromal versican expression by breast cancer cells and importance to relapse-free survival in patients with node-negative primary breast cancer. Clin Cancer Res 8, 1054-60 (2002)). Anti-versican immunoreactivity is increased in the peritumoral stromal matrices of breast (Suwiwat, S. et al., Expression of extra cellular matrix components versican, chondroitin sulfate, tenascin, and hyaluronan, and their association with disease outcome in node-negative breast cancer. Clin Cancer Res 10, 2491-8. (2004)), lung (Pirinen, R. et al., Versican in nonsmall cell lung cancer: relation to hyaluronan, clinicopathologic factors, and prognosis. Hum Pathol 36, 44-50 (2005)), prostate (Ricciardelli, C. et al., Elevated levels of versican but not decorin predict disease progression in early-stage prostate cancer. Clin Cancer Res 4, 963-71 (1998)), and colon (Mukaratirwa, S. et al., Versican and hyaluronan expression in canine colonic adenomas and carcinomas: relation to malignancy and depth of tumour invasion. J Comp Pathol 131, 259-70 (2004)) cancers. In addition, increased accumulation of versican in the stroma surrounding breast cancer cells is associated with a higher risk of relapse in node-negative, primary breast cancer (Ricciardelli, C. et al. Regulation of stromal versican expression by breast cancer cells and importance to relapse-free survival in patients with node-negative primary breast cancer. Clin Cancer Res 8, 1054-60 . (2002); Suwiwat, S. et al., Expression of extra cellular matrix components versican, chondroitin sulfate, tenascin, and hyaluronan, and their association with disease outcome in node-negative breast cancer. Clin Cancer Res 10, 2491-8 (2004)). Versican was identified from several peptides (up to 5), and MS spectra of versican peptides were thoroughly examinated (Fig. 4b). Further validation of this potential target was undertaken using immunohistochemistry. Fig. 3 shows representative examples of anti-versican immunostaining in human breast tissues. While versican expression around normal breast glands and ducts was generally absent, the protein harbored strong immunoreactivity in the stromal compartment of the 10 different breast cancer lesions analyzed. These results as well as those from the above-referenced literature are thus fully consistent with the mass spectrometry analysis of the present studies detecting versican only in neoplastic tissues. In view of all these data, versican is proposed as a potential target protein for the treatment of human breast cancer. However, an ideal target protein should not only be specifically expressed in tumors, but also be absent in normal tissues. Since the distribution of versican expression in the normal human body was largely unknown, the present inventors undertook to examine in detail the presence of this ECM proteoglycan using tissue microarrays comprising a wide variety of normal human tissues and organs. Analysis of versican expression by immunoperoxidase straining revealed that anti-versican immunoreactivity was absent in most normal tissues (e.g. adrenal gland, amniotic membrane, breast, umbilical cord, endometrium, myometrium, uterine cervix, heart, kidney, oesophagus, pancreas, peripheral nerve, liver, lung, spleen, thymus, thyroid, tongue), with only moderate reactivity detected in the placenta and some areas of the central nervous system. Since the central nervous system is not accessible to circulating, conjugated antibodies unless blood brain barrier is disrupted, these *in situ* expression analyses further identified versican as a promising target for antibody-based anti-cancer treatments.

Periostin is a soluble, secreted ECM-associated protein (Takeshita, S. et al., Osteoblast-specific factor 2: cloning of a putative bone adhesion protein with homology with the insect protein fasciclin I. Biochem J 294 ( Pt 1), 271-8 (1993))that localizes with integrins at sites of focal adhesion, thus suggesting a contribution of this protein to cell adhesion and motility (Gillan, L. et al. Periostin secreted by epithelial ovarian carcinoma is a ligand for alpha(V)beta(3) and alpha(V)beta(5) integrins and promotes cell motility. Cancer Res 62, 5358-64 (2002)). It has been recently shown that human periostin is overexpressed in several cancer types, including colorectal carcinoma (Bao, S. et al., Periostin potently promotes metastatic growth of colon cancer by augmenting cell survival via the Akt/PKB pathway. Cancer Cell 5, 329-39 (2004); Tai, I. T. et al., Periostin induction in tumor cell line explants and inhibition of in vitro cell growth by anti-periostin antibodies. Carcinogenesis 26, 908-15 (2005)), breast adenocarcinoma (Shao, R. et al., Acquired expression of periostin by human breast cancers promotes tumor angiogenesis through up-regulation of vascular endothelial growth factor receptor 2 expression. Mol Cell Biol 24, 3992-4003 (2004)), non-small cell lung carcinoma (Sasaki, H. et al., Expression of Periostin, homologous with an insect cell adhesion molecule, as a prognostic marker in non-small cell lung cancers. Jpn J Cancer Res 92, 869-73 (2001)), glioblastoma (Lal, A. et al., A public database for gene expression in human cancers. Cancer Res 59, 5403-7 (1999)), and epithelial ovarian carcinoma (Ismail, R. S. et al., Differential gene expression between normal and tumor-derived ovarian epithelial cells. Cancer Res 60, 6744-9 (2000)). In addition, periostin overexpression in cancer lesions may be associated with advanced disease (Bao, S. et al., Periostin potently promotes metastatic growth of colon cancer by augmenting cell survival via the Akt/PKB pathway. Cancer Cell 5, 329-39 (2004)) and poor outcome (Sasaki, H. et al., Expression of Periostin, homologous with an insect cell adhesion molecule, as a prognostic marker in non-small cell lung cancers. Jpn J Cancer Res 92, 869-73 (2001)). Periostin may facilitate tumor invasion and metastasis by promoting angiogenesis (Bao, S. et al., Periostin potently promotes metastatic growth of colon cancer by augmenting cell survival via the Akt/PKB pathway. Cancer Cell 5, 329-39 (2004); Shao, R. et al., Acquired expression of periostin by human breast cancers promotes tumor angiogenesis through up-regulation of vascular endothelial growth factor receptor 2 expression. Mol Cell Biol 24, 3992-4003 (2004)). Given the suspected role of periostin in tumor metastasis, this secreted protein represents an attractive cancer biological marker and target. In the current study, protein expression profiles obtained in the 10 breast cancer and matched non-tumoral tissues analyzed revealed that periostin was among the most frequently extra cellular proteins identified in the tumor samples (10 out of the 10 breast cancers evaluated). This was further confirmed by immunohistochemical experiments demonstrating an up-regulation of periostin in the stroma associated with the breast cancers analyzed in this study, as compared with the stroma associated with the non-tumoral breast glandular/ductal compartment (data not shown).

**3. Figure legends**

Figure 1 shows a schematic representation of a method for the identification of accessible proteins in human pathologic tissues. *Ex vivo* biotinylation of normal and diseased human tissue samples is achieved by immersion of the tissues into a solution containing a reactive ester derivative of biotin that labels free primary amines. Biotinylated proteins from each tissue sample are extracted with detergents, captured on streptavidin, and submitted to on-resin proteolytic digestion after washes. Biotinylated accessible proteins are finally sequenced by shotgun mass spectrometry using nLC-ESI MS/MS. Identified accessible proteins differentially expressed in the tissue samples are further validated for example by immunohistochemical analysis.

Figure 2 shows the evaluation of increasing immersion times on the depth of tissue biotinylation. Thin slices of breast cancer tissues were soaked in the biotinylation solution for various periods of time, as indicated. Extent of tissue biotinylation was assessed using histochemistry. Histochemical experiments were carried out with the use of the ABC Vectastain kit, according to the manufacturer's directions (Vector Laboratories, Burlingame, CA, USA). Original magnification: x100.

Figure 3: Validation of versican by *in situ* expression analyses. a: Representative examples of versican expression in cancerous and matched non-tumoral human breast cancer tissues, as assessed by immunohistochemistry. Paraffin sections of human breast tissues were subjected to immunoperoxidase staining. Original magnification: x100. b: Representative examples of versican expression by immunoperoxidase staining in various normal human tissues and organs, as indicated, using tissue microarrays.

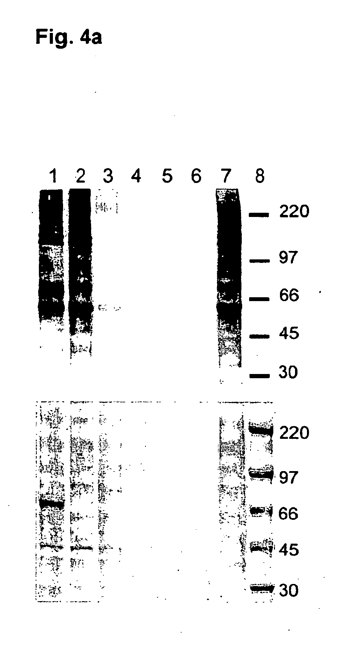

Figure 4: Sample processing overview. a: Purification of biotinylated proteins. The upper blot shows biotinylated proteins from the coomassie blue (CB) stained gel depicted in the lower panel. Whole, tumor lysate is shown in lane 1. Human serum albumin and immunoglobulins are removed from the samples, as clearly shown in lane 2 (CB gel): This lysate is applied onto a streptavidin resin. Lane 3 shows proteins that did not attach to the resin: a significant amount of protein did not attach, but few proteins were actually biotinylated. Lanes 4, 5 and 6 show the flow-through fractions of different washes (with detergent, high salt and alkaline buffer, respectively). Proteins attached to the resin are shown in lane 7; these proteins represent those that are on-resin digested and analysed by MS. b: MS/MS fragmentation of the peptide LLASDAGLYR found in the versican core protein precursor, followed by MS/MS fragmentation annotated by Mascot software. The monoisotopic mass of the neutral peptide is 1077.58, the measured mass is 1077.535448 (from the doubly charged parent ion 539.775000). The table below the fragmentation spectra shows ions from the b and y series (20 out of 74 fragment ions using the 37 most intense peaks). *m*/*z*: mass-to-charge ratio. c: Reproducibility of the biotinylation steps and MS analyses. A breast tumor specimen was cut into four samplers, which were biotinylated separately. Each sample was stained by histochemistry with avidin-HRP conjugates as shown for sample n°4 (original magnification: x100). The patterns of biotinylated proteins from the first, second and third samples were revealed by neutravidin-HRP after western blotting. Lane 3 shows a weaker staining, but an overall identical pattern when compared with lanes 1 and 2. Sample n°1 was analysed by MS 3 times. The histograms on the left side of the figure display the average number of proteins identified following 1, 2 or 3 replicate analyses of the same sample (error bars indicate the standard error to the mean for each pairwise replicate comparison), and the overlap of protein identified in the three replicate runs. Samples n°1, 2 and 3 were analysed by MS. The average number of identifications and the percentage of overlap in protein identifications are reported in the histograms.

**4. Tissue harvesting**

Cancerous and non-tumoral human breast tissue samples were obtained from mastectomy specimens, immediately sliced and soaked into freshly prepared biotinylation solution (1 mg.ml⁻¹ of EZ-link Sulfo NHS-LC biotin (Pierce) dissolved extemporaneously in PBS [pH 7.4]). The time frame from the excision in the operating theatre to the biotinylation step required typically less than 10 minutes. Each biotinylation reaction was stopped by a 5 minute incubation in a primary amine-containing buffer solution (e.g. 50mM Tris [pH 7.4]). Tissue samples were then snap-frozen in liquid nitrogen, except for a tiny portion of each sample that was directly immersed in formalin and then processed for further histological and histochemical investigations. Additional tissue samples not included in the biotinylation procedure were routinely processed for histopathological diagnosis. Controls included adjacent tissue slices immersed in PBS. The Ethics Committee of the University Hospital of Liege reviewed and approved the specific protocol used in this study, and written informed consent was obtained from all patients.

**5. Histochemistry and immunohistochemistry**

In order to assess the diffusion depth of reactive biotin ester derivatives in the biotinylated tissues, formalin-fixed, paraffin-embedded breast tissue sections were incubated with avidin-peroxidase conjugates with the use of the Vectastain ABC kit (Vector Labortories, Burlingame, CA, USA), according to the manufacturer's instructions. Immunohistochemical experiments were performed as previously described by Waltregny et al. (Waltregny, D. et al. Prognostic value of bone sialoprotein expression in clinically localized human prostate cancer. J Natl Cancer Inst 90, 1000-1008 (1998)). For the immunohistochemical detection of versican, antigen retrieval was performed by incubating the slides with chondroitinase. Anti-versican (clone 12C5, Developmental Studies Hybridoma Bank at the University of lowa, lowa City, lA, USA) antibody was applied onto the sections at a dilution of 1:200. Control experiments included omission of the primary antibody in the procedure.

**6. Sample processing**

Pulverization of frozen biotinylated biopsies was performed using a Mikro-Dismembrator U (Braun Biotech, Melsungen, Germany) and generated tissue powder that was resuspended first in a PBS buffer containing a protease inhibitor cocktail (Complete, Roche Diagnostics, Mannheim, Germany). Homogenates were sonicated (2x30") with a 2mm microprobe and soluble proteins were subjected to a preclearing step consisting in human serum albumin (HSA) and immunoglobulins (IgGs) depletion (Qproteome HSA and IgGs Removal Kit, Quiagen). This step was included to limit the number of HSA and IgGs peptides detected by MS, but did not hinder detection of low abundant proteins (data not shown). Insoluble pellet' was resuspended in 2% SDS in PBS, and lysates were sonicated (3x30"). HSA- and IgGs-depleted soluble proteins fraction and detergent-solubilized proteins were pooled and boiled for 5 minutes. Protein concentration was determined using the BCA protein assay reagent kit (Pierce Chemical Co.). Streptavidin-sepharose slurry (Amersham Biosciences, 150µl per mg of total proteins) was equilibrated by three washes in buffer A (1 % NP40 and 0.1% SDS in PBS), and protein binding was allowed for 2 hours at room temperature in a rotating mixer. The resin was then washed twice with buffer A, twice with buffer B (0.1% NP40, 1M NaCl in PBS), twice with buffer C (0.1M podium carbonate in PBS, pH 11), and once with ammonium hydrogenocarbonate (50mM, pH 7.8). Binding of the biotinylated proteins onto the resin and washing efficiencies were checked by SDS-PAGE, and further either by Coomassie blue straining or by blotting for subsequent detection of biotin by streptavidin-horseradish peroxidase. On-resin digestion was carried out overnight at 37°C with agitation, using modified porcine trypsin (Promega) in 100µl final volume of ammonium hydrogenocarbonate (pH 7.8). The supernatants were collected, protein concentration was determined, and once evaporated, the peptides were resuspended in 0.1% formic acid. Samples were analysed by nanocapillary liquid chromatography-electrospray tandem mass spectrometry (nLC-ESI MS/MS).

**7. Mass spectrometry**

Peptide separation by reverse-phase liquid chromatography was performed on an "Ultimate LC" system (LC Packings) completed with a Famos autosampler and a Swichos II Microcolumn switching device for sample clean-up, fractionation and preconcentration. Sample (20µL at 0.25µg/µL 0.1% formic acid) was first trapped on a SCX micro pre-column (500µM internal diameter, 15mm length, packed with MCA50 bioX-SCX 5µm; LC Packings) at a flow rate of 200nL/min followed by a micro pre-column cartridge (300µM i.d., 5mm length, packed with 5µm C18 PepMap100 ; LC Packings). After 5 min the precolumn was connected with the separating nanocolumn (75µm i.d., 15cm length, packed with 3µm C18 PepMAp100 ; LC Packings) equilibrated in mobile phase A (0,1% formic acid in 2:98 of acetonitrile:degassed milliQ water). A linear elution gradient was applied with mobile phase B (0.1% formic acid in 80:20 of acetonitrile:degassed milliQ water) from 10% to 40% spanning on 95 minutes. The outlet of the LC system was directly connected to the nano electrospray source of an Esquire HCT ion trap mass spectrometer (Bruker Daltonics, Germany). Mass data acquisition was performed in the mass range of 50-2000 m/z using the standard-enhanced mode (8100 m/z per second). For each mass scan, a data dependant scheme picked the 3 most intense doubly or triply charged ions to be selectively isolated and fragmented in the trap. The resulting fragments were analyzed using the Ultra Scan mode (m/z range of 50-3000 at 26000m/z per second). SCX-trapped peptides were stepwise eluted with 5 salt concentrations (10mM, 20mM, 40mM, 80mM, and 200mM), each followed by the same gradient of mobile phase B.

**8. Data processing and mgf file generation**

Raw spectra Were formatted in DataAnalysis software (Bruker Daltonics). Portion of the chromatogram containing signal (i.e. with base peak chromatogram signal above 50000 arbitrary units) was processed to extract and deconvolute MS/MS spectra, without smoothing or background substraction. A signal/noise ratio of 3 was applied to filtrate irrelevant data in the MS/MS spectra and generate the mass list. Charge deconvolution was performed on both MS and MS/MS spectra. A retention time of 1.5 minutes was allowed for compound elution to minimise detection redundancy of parents of identical masses and charge states. Both deconvoluted and undeconvoluted data were incorporated in the mgf file.

**9. Database Searching**

Protein identification was performed using different databases and different softwares featuring different built-in algorithms. Protein were first identified using the NCBI nonredundant database (NCBlnr, release 20060131) through the Phenyx web interface (GeneBio, Geneva, Switzerland). The mass tolerance of precursor ions was set at 0.6; allowed modifications were partial oxidization of methionines and partial lysin-modifications with LC biotin. One misscut was also allowed. For each tumor samples, identification of more than 400 proteins were obtained. Additional searches were performed against the minimally redundant SWISS-PROT, human protein database (Nesvizhskii, A. I. & Aebersold, R. Interpretation of Shotgun Proteomic Data: The Protein Inference Problem. Mol Cell Proteomics 4, 1419-1440 (2005)) through the MS/MS ion search algorithm of the Mascot search engine (Mascot and Mascot Daemon v2.1.0) (Perkins, D. N. et al., Electrophoresis 20, 3551-67 (1999)) running on a multi-processor computer cluster. The mass tolerance of precursor and fragmented ions were set at 0.6 and 0.3 respectively; allowed modifications were partial oxidization of methionines. Stringent filtration was deliberately used: the absolute probability (P) was set to 0.01 (i.e. less than 1% probability of a random match), and the MudPIT scoring was used, while discriminating peptides with ion scores inferior to 15. Despite this "aggressive" filtering, proteins hits were manually inspected, particularly for those proteins identified by only one peptide. These precautions were taken to ascertain the accuracy of protein identification reported in Table 1. Proteins of interest were identified by both Phenyx and Mascot search engines, the sequence coverage being usually higher with Phenyx (data not shown).

**10. Tissue microarrays.**

Medium-density tissue microarrays (TMAs) comprising 0.6mm cores of various normal human tissues were assembled as previously described (Kononen, J. et al. (1998) Tissue microarrays for high-throughput molecular profiling of tumor specimens. Nat Med 4, 844-847; Perrone, E. E. et al. (2000) Tissue Microarray Assessment of Prostate Cancer Tumor Proliferation in African-American and White Men. J Natl Cancer Inst 92, 937-939). Formalin-fixed, paraffin-embedded blocks of normal human tissues were retrieved from the Department of Pathology of the University Hospital of Liege. Most normal tissues were from surgical specimens, except for neuronal tissues, which were obtained from autopsies. Initial sections were stained with haematoxylin and eosin to verify histology. Two TMAs were generated with the use of a manual tissue arrayer (Beecham Instruments). Duplicate cores were included in the TMA for each specific tissue or organ analysed. A total of 120 cores were examined for immunohistochemical expression of versican. Immunostaining intensity was scored as absent, weak, moderate or strong by 2 observers.

**Table 1: Selection of proteins identified in the breast tumors (n=10) and associated adjacent normal breast tissues (n=10). The proteins are identified from the SwissProt database (release 49.5) with Mascot (v2.1). The numbers indicate in how many breast samples each protein was detected. Location of the proteins was determined using both the Human Protein Reference Database (www.hprd.org) and Bioinformatic Harvester (harvester.embl.de). C: Cytoplasmic, E: Extracellular, ER: Endoplasmic Reticulum, Mb: plasma membrane, N: Nuclear.**

| Swiss Prot # | Protein Name | Sequence coverage (lowest-highest) | MudPIT Score (lowest-highest) | Location | Non Tumoral Breast | Tumoral Breast |
|---|---|---|---|---|---|---|
| P13611 | Versican core protein precursor | 0.2%-1.5% | 40-221 | E | 0 | 10 |
| P08727 | Keratin, type I cytoskeletal 19 | 10-55% | 212-1270 | C | 1 | 10 |
| P02751 | Fibronectin precursor | 0.7-12% | 56-3688 | E | 3 | 10 |
| Q15063 | Periostin precursor | 1-26% | 60-3383 | E | 5 | 10 |
| P12111 | Collagen type VI alpha 3 | 7-26% | 73-573 | E | 10 | 10 |
| P51884 | Lumican precursor | 13-33% | 313-2009 | E | 10 | 10 |
| P05783 | Keratin, type I cytoskeletal 18 | 4-25% | 49-543 | C,N | 0 | 8 |
| 095994 | Anterior gradient protein 2 homolog precursor | 6-42% | 39-775 | ER | 1 | 7 |
| P24821 | Tenascin precursor | 1-11% | 41-1471 | E, Mb | 1 | 5 |
| P02533 | Keratin, type I cytoskeletal 14 | 3-17% | 75-417 | C | 5 | 1 |

**Table 2: Clinico-pathological characteristics of the breast carcinomas analyzed in the study. Cancerous and matched non-tumoral tissues from each mastectomy specimen were soaked in PBS or EZ-link Sulfo-NHS-LC-biotin, and then flash frozen in liquid nitrogen for subsequent analyses. The grading system used is based on the Bloom classification modified by Elston. Estrogen receptor (ER), progesterone receptor (PR), and ErbB2 status of the breast cancer lesions was routinely assessed by immunohistochemistry.**

| # | Age | Tumor size (cm) | Type | Grade | Nodal status | ER | PR | ErbB2 |
|---|---|---|---|---|---|---|---|---|
| 1 | 74 | 2.5 | Infiltrating ductal | 2 | N0 | + | + | - |
| 2 | 56 | 4 | Infiltrating lobular | 2 | N0 | + | + | - |
| 3 | 56 | 1.8 | Infiltrating ductal | 3 | N+ | + | + | - |
| 4 | 74 | 1.5 | Infiltrating lobular | 2 | N0 | + | - | - |
| 5 | 78 | 2.2 | Infiltrating ductal | 3 | N0 | - | - | + |
| 6 | 74 | 2.5 | Infiltrating lobular | 2 | N0 | + | - | - |
| 7 | 61 | 1.7 | Infiltrating ductal | 3 | N0 | - | - | - |
| 8 | 77 | 2.8 | Infiltrating ductal | 3 | N0 | - | - | - |
| 9 | 80 | 2.2 | Infiltrating ductal | 2 | N0 | + | + | - |
| 10 | 73 | 1.5 | Infiltrating ductal | 3 | N+ | + | - | - |

**Table 3: Complete list of proteins identified by nanoLC-ESI-MS/MS analysis with stringent parameters. The numbers indicate the number of patients for which proteins were found either in cancerous or in non-tumoral tissues. The proteins were identified from the SwissProt database with Mascot using stringent parameters (p<0.01). The numbers indicate in how many breast samples each protein was detected. Location of the proteins was determined using the Human Protein Reference Database (www.hprd.org) and Bioinformatic Harvester (harvester.embl.de). C: Cytoplasmic, E: Extracellular, Mb: plasma membrane, N: Nuclear, U: Unknown.**

| **Protein Name** | **Swiss Prot #** | **Location** | **Non Tumoral Breast** | **Tumoral Breast** |
|---|---|---|---|---|
| **Versican core protein precursor** | P13611 | E | **0** | **10** |
| **Keratin, type I cytoskeletal 18** | P05783 | C, N | **0** | **8** |
| **Rho GDP-dissociation inhibitor 2** | P52566 | C | **0** | **7** |
| **Calreticulin precursor** | P27797 | C, E, Mb | **0** | **6** |
| **Annexin A6** | P08133 | C, Mb | **0** | **6** |
| **Calnexin precursor** | P27824 | C, Mb | **0** | **6** |
| **Talin-1** | Q9Y490 | E, Mb, C | **0** | **6** |
| **Heterogeneous nuclear ribonucleoprotein D0** | Q14103 | C | **0** | **5** |
| **lsocitrate dehydrogenase** | P48735 | C | **0** | **5** |
| **ATP-dependent DNA helicase 2 subunit 2** | P13010 | N | **0** | **5** |
| **Heterogeneous nuclear ribonucleoprotein A1** | P09651 | N, C | **0** | **5** |
| **Heterogeneous nuclear ribonucleoproteins A2/B1** | P22626 | N, C | **0** | **5** |
| **Macrophage migration inhibitory factor** | P14174 | E, C | **0** | **5** |
| **Tropomyosin alpha 4 chain** | P67936 | C | **0** | **4** |
| **Myosin regulatory light chain 2** | P19105 | C | **0** | **4** |
| **Actin-like protein 2** | P61160 | C | **0** | **4** |
| **Elongation factor 1-delta** | P29692 | C | **0** | **4** |
| **Peroxiredoxin 5** | P30044 | C | **0** | **4** |
| **Malate dehydrogenase mitochondrial precursor** | P40926 | C | **0** | **4** |
| **Nucleophosmin** | P06748 | N, C | **0** | **4** |
| **Heterogeneous nuclear ribonucleoprotein K** | P61978 | N, C | **0** | **4** |
| **Adenylyl cyclase-associated protein 1 (CAP 1)** | Q01518 | Mb | **0** | **4** |
| **ATP-dependent RNA helicase A** | Q08211 | C, N | **0** | **3** |
| **Cellular retinoic acid-binding protein 2** | P29373 | C | **0** | **3** |
| **Vitamin B** | 075369 | C | **0** | **3** |
| **Isocitrate dehydrogenase** | 075874 | C | **0** | **3** |
| **Caidesmon** | Q05682 | C, Mb | **0** | **3** |
| **Signal transducer and activator of transcription 1-alpha/beta** | P42224 | C, N | **0** | **3** |
| **LIM and SH3 domain protein 1** | Q14847 | C | **0** | **3** |
| **Calmodulin (CaM)** | P62158 | C, N, Mb | **0** | **3** |
| **Ras-related protein Rab-1B** | Q9H0U4 | C | **0** | **3** |
| **Thrombospondin-1 precursor** | P07996 | E | **0** | **3** |
| **Prohibitin** | P35232 | C, Mb, E | **0** | **3** |
| **Heterogeneous nuclear ribonucleoprotein A3** | P51991 | N, C | **0** | **3** |
| **ATP-dependent DNA helicase 2 subunit 1** | P12956 | N, C | **0** | **3** |
| **Interleukin enhancer-binding factor 2** | Q12905 | N | **0** | **3** |
| **Sodium/potassium-transporting ATPase α-1 chain precursor** | P05023 | Mb | **0** | **3** |
| **Tubulin beta-3 chain** | Q13509 | C | **0** | **3** |
| **Superoxide dismutase mitochondrial precursor** | P04179 | C | **0** | **3** |
| **Major vault protein** | Q14764 | C, N | **0** | **2** |
| **Coronin-1A** | P31146 | C | **0** | **2** |
| **Cytosolic nonspecific dipeptidase** | Q96KP4 | C | **0** | **2** |
| **60S ribosomal protein L15** | P61313 | C | **0** | **2** |
| **40S ribosomal protein S4, 1 isoform** | P62701 | C | **0** | **2** |
| **T-complex protein 1 subunit theta** | P50990 | C | **0** | **2** |
| **Hsc70-interacting protein** | P50502 | C | **0** | **2** |
| **Coatomer subunit alpha** | P53621 | C | **0** | **2** |
| **Protein transport protein Sec23A** | Q15436 | C | **0** | **2** |
| **60S ribosomal protein L9** | P32969 | C | **0** | **2** |
| **Aspartyl-tRNA synthetase** | P14868 | C | **0** | **2** |
| **T-complex protein 1 subunit eta** | Q99832 | C | **0** | **2** |
| **Peroxiredoxin 6** | P30041 | C | **0** | **2** |
| **Transaldolase** | P37837 | C | **0** | **2** |
| **T-plastin** | P13797 | C | **0** | **2** |
| **Adenosylhomocysteinase** | P23526 | C | **0** | **2** |
| **Glutathione S-transferase Mu 1** | P09488 | C | **0** | **2** |
| **Elongation factor 1-bet** | P24534 | C | **0** | **2** |
| **Carbonic anhydrase 2** | P00918 | C, Mb, N | **0** | **2** |
| **Legumain precursor** | Q99538 | C | **0** | **2** |
| **NADH-cytochrome b5 reductase** | P00387 | C, Mb, C | **0** | **2** |
| **Coatomer subunit beta'** | P35606 | C | **0** | **2** |
| **Trifunctional purine biosynthetic protein adenosine-3** | P22102 | C | **0** | **2** |
| **Coatomer subunit beta** | P53618 | C | **0** | **2** |
| **Coatomer subunit gamma-2** | Q9UBF2 | C | **0** | **2** |
| **Tubulin alpha-3 chain** | Q71 U36 | C | **0** | **2** |
| **Transmembrane emp24 domain-containing protein 9 precursor** | Q9BVK6 | C | **0** | **2** |
| **Ras-related protein Rab-1A** | P62820 | C | **0** | **2** |
| **Surfeit locus protein 4** | 015260 | C | **0** | **2'** |
| **Galectin-1** | P09382 | E, C, Mb | **0** | **2** |
| **EMILIN-1 precursor** | Q9Y6C2 | E, C | **0** | **2** |
| **Ig kappa chain V-I region DEE** | P01597 | E | **0** | **2** |
| **Apolipoprotein A-II precursor** | P02652 | E | **0** | **2** |
| **Galectin-3-binding protein precursor** | Q08380 | E, Mb | **0** | **2** |
| **Collagen type I alpha 1** | P02452 | E | **0** | **2** |
| **Lysosomal alpha-glucosidase precursor** | P10253 | C | **0** | **2** |
| **Voltage-dependent anion-selective channel protein 3** | Q9Y277 | C | **0** | **2** |
| **Elongation factor Tu, mitochondrial precursor** | P49411 | C | **0** | **2** |
| **Cytochrome C oxidase polypeptide VIIa-liver/heart, precursor** | P14406 | C | **0** | **2** |
| **Ubiquinol-cytochrome-c reductase complex core protein 2** | P22695 | C | **0** | **2** |
| **Programmed cell death protein 8, mitochondrial precursor** | O95831 | C, N | **0** | **2** |
| **Heterogeneous nuclear ribonucleoprotein C-like** | 060812 | N, C | **0** | **2** |
| **Histone H2B** | P33778 | N | **0** | **2** |
| **Poly(rC)-binding protein 1** | Q15365 | N | **0** | **2** |
| **Lamin B2** | Q03252 | N | **0** | **2** |
| **Splicing factor, proline- and glutamine-rich** | P23246 | N, Mb | **0** | **2** |
| **ATP-dependent RNA helicase DD139** | 000148 | N | **0** | **2** |
| **Ras-related protein Rab-11.8** | Q15907 | N, C, Mb | **0** | **2** |
| **ATP-dependent RNA helicase DD11** | Q92499 | N | **0** | **2** |
| **Heterogeneous nuclear ribonucleoprotein H** | P31943 | N, C | **0** | **2** |
| **HLA class I histocompatibility antigen, B-15 α-chain precursor** | P30464 | Mb | **0** | **2** |
| **HLA class II histocompatibility antigen** | P01903 | Mb, C | **0** | **2** |
| **HLA class I histocompatibility antigen** | P01893 | Mb | **0** | **2** |
| **HLA class II histocompatibility antigen, gamma chain** | P04233 | Mb, C | **0** | **2** |
| **SPFH domain-containing protein 1 precursor** | 075477 | Mb | **0** | **2** |
| **Myoferlin** | Q9NZM1 | Mb | **0** | **2** |
| **Thy-1 membrane glycoprotein precursor** | P04216 | Mb | **0** | **2** |
| **Twinfilin-1** | Q12792 | U | **0** | **2** |
| **Heat shock 70 kDa protein 4** | P34932 | U | **0** | **2** |
| **Protein FAM49B (L1)** | Q9NUQ9 | U | **0** | **2** |
| **Receptor tyrosine-protein kinase ErbB2 precursor** | P04626 | Mb, E | **0** | **1** |
| **6S proteasome non-ATPase regulatory subunit 2** | Q13200 | C | **0** | **1** |
| **ATP-citrate synthase** | P53396 | C | **0** | **1** |
| **Hexokinase-1** | P19367 | C, Mb, C | **0** | **1** |
| **Interferon-induced GTP-binding protein Mx1** | P20591 | C | **0** | **1** |
| **AICAR transformylase, IMP cyclohydrolase** | P31939 | C | **0** | **1** |
| **Septin-9** | Q9UHD8 | C, Mb | **0** | **1** |
| **40S ribosomal protein S16** | P62249 | C | **0** | **1** |
| **Integrin-linked protein kinase 1** | Q13418 | C | **0** | **1** |
| **605 ribosomal protein L27** | P61353 | C | **0** | **1** |
| **6-phosphofructokinase, liver type** | P17858 | C | **0** | **1** |
| **40S ribosomal protein S9** | P46781 | C | **0** | **1** |
| **Biliverdin reductase A precursor** | P53004 | C | **0** | **1** |
| **Puromycin-sensitive aminopeptidase** | P55786 | C, N | **0** | **1** |
| **Eukaryotic translation initiation factor 4 gamma 1 Kinectin** | Q04637 Q86UP2 | C | **0** | **1** |
| **Breast cancer- associated protein SGA-113M** | Q15008 | C | **0** | **1** |
| **Elongation factor 1-gamma** | P26641 | C | **0** | **1** |
| **Copine-3** | 075131 | C | **0** | **1** |
| **Vesicle-fusing. ATPase** | P46459 | C | **0** | **1** |
| **605 ribosomal protein L10a** | P62906 | c | **0** | **1** |
| **60S ribosomal protein L22** | P35268 | C | **0** | **1** |
| **Cysteine and glycine-rich protein 1** | P21291 | C, N | **0** | **1** |
| **40S ribosomal protein S8** | P62241 | C | **0** | **1** |
| **40S ribosomal protein S6** | P62753 | C | **0** | **1** |
| P**roteasome subunit alpha type 4** | P25789 | C, N | **0** | **1** |
| **Fructose-bisphosphate aldolase C** | P09972 | C | **0** | **1** |
| **40S ribosomal protein S2** | P15880 | C | **0** | **1** |
| **T-complex protein 1 subunit alpha** | P17987 | C | **0** | **1** |
| **Kynureninase** | Q16719 | C | **0** | **1** |
| **Ras-related protein Rab-7** | P51149 | C | **0** | **1** |
| **T-complex protein 1 subunit zeta** | P40227 | C | **0** | **1** |
| **Eukaryotic translation initiation factor 1** | P41567 | C | **0** | **1** |
| **Calpastatin** | P20810 | C, N | **0** | **1** |
| **Toll-interacting protein** | Q9H0E2 | C | **0** | **1** |
| **FK506-binding protein 4** | Q02790 | C, N | **0** | **1** |
| **Actin-related protein 2/3 complex subunit 4** | P59998 | C | **0** | **1** |
| **40S ribosomal protein S28** | P62857 | C, N | **0** | **1** |
| **General vesicular transport factor p115** | 060763 | C, N | **0** | **1** |
| **40S ribosomal protein SA** | P08865 | C, N | **0** | **1** |
| **Actin-related protein 2/3 complex subunit 3** | O15145 | C | **0** | **1** |
| **Dihydropyrimidinase-related protein 3** | Q14195 | C | **0** | **1** |
| **Proteasome subunit alpha type 2** | P25787 | C, N | **0** | **1** |
| **Fructose-1,6-bisphosphatase 1** | P09467 | C, N | | **1** |
| **Coactosin-like protein** | Q14019 | C | **0** | **1** |
| **Dynein heavy chain** | Q14204 | C, Mb | **0** | **1** |
| **Ras-related protein Rab-35** | Q15286 | C | **0** | **1** |
| **Keratin, type I cytoskeletal 15** | P19012 | C | **0** | **1** |
| **Cytoplasmic dynein 1 intermediate chain 2** | Q13409 | C | **0** | **1** |
| **Malate dehydrogenase, cytoplasmic** | P40925 | C | **0** | **1** |
| **Lactoylglutathione lyase** | Q04760 | C | **0** | **1** |
| **60S ribosomal protein L14** | P50914 | C | **0** | **1** |
| **Desmoplakin** | P15924 | C, Mb, N | **0** | **1** |
| **Nucleoside diphosphate kinase A** | P15531 | C, N | **0** | **1** |
| **Glutathione S-transferase Mu 3** | P21266 | C | **0** | **1** |
| **Tubulin alpha-6 chain** | Q9BQE3 | C | **0** | **1** |
| **Keratin, type II cytoskeletal 6.B** | P04259 | C | **0** | **1** |
| **40S ribosomal protein S14** | P62263 | C | **0** | **1** |
| **Coatomer subunit delta** | P48444 | C | **0** | **1** |
| **Sorting nexin-9** | Q9Y511 | C, Mb | **0** | **1** |
| **Rab GDP dissociation inhibitor alpha** | P31150 | C | **0** | **1** |
| **Bifunctional aminoacyl-tRNA synthetase** | P07814 | C | **0** | **1** |
| **Ferritin heavy chain** | P02794 | C | **0** | **1** |
| **F-actin capping protein alpha-2 subunit** | P47755 | C | **0** | **1** |
| **Eukaryotic translation initiation factor 6** | P56537 | C, N | **0** | **1** |
| **Signal recognition particle 14 kDa protein** | P37108 | C | **0** | **1** |
| **60S ribosomal protein L27a** | P46776 | C | **0** | **1** |
| **Translin-associated protein 1** | Q99598 | C, N | **0** | **1** |
| **Proteasome subunit beta type 1** | P20618 | C | **o** | **1** |
| **Serine hydroxymethyltransferase, cytosolic** | P34896 | C | **0** | **1** |
| **Nicotinamide phosphoribosyltransferase** | P43490 | C | **0** | **1** |
| **lmportin beta-1 subunit** | Q14974 | C, N | **0** | **1** |
| **14-3-3 protein theta (14-3-3 protein tau)** | P27348 | C, N | **0** | **1** |
| **Acid ceramidase precursor** | Q13510 | C | **0** | **1** |
| **Phosphoglucomutase-1** | P36871 | C | **0** | **1** |
| **Tubulin-specific chaperone B** | Q99426 | C | **0** | **1** |
| **Platelet-activating factor acetylhydrolase IB gamma subunit** | Q15102 | C | **0** | **1** |
| **lmportin beta-3** | 000410 | C, N | **0** | **1** |
| **Ras GTPase-activating-like protein IQGAP2** | Q13576 | C | **0** | **1** |
| **Dipeptidyl-peptidase 2 precursor** | Q9UHL4 | C | **0** | **1** |
| **N(4)-(beta-N-acetylglucosaminyl)-L-asparaginase precursor** | P20933, | C | **0** | **1** |
| **26S proteasome non-ATPase regulatory subunit 12** | 000232 | C | **0** | **1** |
| **Putative eukaryotic translation initiation factor 3 subunit** | 075153 | C | **0** | **1** |
| **Inositol monophosphatase** | P29218 | C | **0** | **1** |
| **60S ribosomal protein L7a** | P62424 | C | **0** | **1** |
| **AP-2 complex subunit mu-1** | Q96CW1 | C | **0** | **1** |
| **T-complex protein 1 subunit delta** | P50991 | C | **0** | **1** |
| **UTP-glucose-1-phosphate uridylyltransferase 2** | Q16851 | C | **0** | **1** |
| **Translocon-associated protein gamma subunit** | Q9UNL2 | C, N | **0** | **1** |
| **60S ribosomal protein L11** | P62913 | C | **0** | **1** |
| **Fumarylacetoacetase** | P16930 | C | **0** | **1** |
| **Probable aminopeptidase NPEPL1** | Q8NDH3 | C | **0** | **1** |
| **Glucosamine--fructose-6-phosphate aminotransferase** | Q06210 | C | **0** | **1** |
| **ADP-ribosylation factor-like 10B** | Q96BM9 | C | **0** | **1** |
| **ADP-ribosylation factor 5** | P84085 | C | **0** | **1** |
| **Thioredoxin domain-containing protein 4 precursor** | Q9BS26 | C | **0** | **1** |
| **Eukaryotic translation initiation factor 2 subunit 3** | P41091 | C | **0** | **1** |
| **Serine/threonine-protein phosphatase PP1-β catalytic subunit** | P62140 | C | **0** | **1** |
| **Ras-related protein Rab-5C** | P51148 | C | **0** | **1** |
| **NAD(P)H dehydrogenase [quinone] 1** | P15559 | C, N | **0** | **1** |
| **6-phosphofructokinase type** | Q01813 | C | **0** | **1** |
| **Septin-2** | Q15019 | C, Mb | **0** | **1** |
| **Superoxide dismutase** | P00441 | C | **0** | **1** |
| **60S acidic ribosomal protein P0** | P05388 | C, N | **0** | **1** |
| **Eukaryotic translation initiation factor 2 subunit 2** | P20042 | C | **0** | **1** |
| **60S ribosomal protein L7** | P18124 | C, N | **0** | **1** |
| **Glycylpeptide N-tetradecanoyltransferase 1** | P30419 | C | **0** | **1** |
| **40S ribosomal protein S25** | P62851 | C | **0** | **1** |
| **Ribonuclease inhibitor** | P13489 | C | **0** | **1** |
| **60S ribosomal protein L4** | P36578 | C | **0** | **1** |
| **GTP-binding nuclear protein Ran** | P62826 | C | **0** | **1** |
| **60S ribosomal protein L5** | P46777 | C, N | **0** | **1** |
| **60S ribosomal protein L24** | P83731 | C | **0** | **1** |
| **Dynamin-1-like protein** | 000429 | C | **0** | **1** |
| **Neutral alpha-glucosidase AB precursor** | Q14697 | C | **0** | **1** |
| **Peptidyl-prolyl cis-trans isomerase C** | P45877 | C, E | **0** | **1** |
| **Translocon-associated protein delta subunit precursor** | P51571 | C | **0** | **1** |
| **Cytochrome P450 2A6** | P11509 | C | **0** | **1** |
| **ERGIC-53 protein precursor** | P49257 | C | **0** | **1** |
| **Protein transport protein Sec24C** | P53992 | C | **0** | **1** |
| **NADPH--cytochrome P450 reductase** | P16435 | C, Mb | **0** | **1** |
| **RER1 protein** | 015258 | C | **0** | **1** |
| **Antigen peptide transporter 2** | Q03519 | C | **0** | **1** |
| **150 kDa oxygen-regulated protein precursor** | Q9Y4L1 | C | **0** | **1** |
| **Transmembrane protein 109 precursor** | Q9BVC6 | C | **0** | **1** |
| **ERO1-like protein alpha precursor** | Q96HE7 | C, Mb | **0** | **1** |
| **Oligosaccharyl transferase 48 kDa subunit** | P39656 | C | **0** | **1** |
| **Endoplasmic reticulum protein ER29 precursor** | P30040 | C | **0** | **1** |
| **Apolipoprotein-L1 precursor** | O14791 | E | **0** | **1** |
| **Kallistatin precursor** | P29622 | E | **0** | **1** |
| **C-type lectin domain family 3 member A precursor** | 075596 | E | **0** | **1** |
| **Calgranulin A** | P05109 | E, Mb, C | **0** | **1** |
| **Ig kappa chain V-I region BAN** | P04430 | E | **0** | **1** |
| **Complement factor I precursor** | P05156 | E, Mb | **0** | **1** |
| **Prothrombin precursor** | P00734 | E | **0** | **1** |
| **Tenascin-1 precursor** | P22105 | E | **0** | **1** |
| **Complement C5 precursor** | P01031 | E | **0** | **1** |
| **Selenoprotein P precursor** | P49908 | E | **0** | **1** |
| **Ig mu heavy chain disease protein (BOT)** | P04220 | E | **0** | **1** |
| **Thymidine phosphorylase precursor** | P19971 | E, C, N | **0** | **1** |
| **MMP-2 (72 kDa type IV collagenase precursor)** | P08253 | E, Mb | **0** | **1** |
| **Thrombospondin-2 precursor** | P35442 | E | **0** | **1** |
| **Ig heavy chain V-III region GAL** | P01781 | E | **0** | **1** |
| **Agrin precursor** | 000468 | E | **0** | **1** |
| **Anterior gradient protein 3 homolog precursor** | Q8TD06 | E, C | **0** | **1** |
| **Ig kappa chain V-I region Wes** | P01611 | E | **0** | **1** |
| **Lipopolysaccharide-binding protein precursor** | P18428 | E, Mb | **0** | **1** |
| **Collagen alpha-1(11) chain precursor** | P12107 | E | **0** | **1** |
| **Ig heavy chain V-III region TIL** | P01765 | E | **0** | **1** |
| **Beta-2-glycoprotein I precursor** | P02749 | E, Mb, C | **0** | **1** |
| **Chymase precursor** | P23946 | E | **0** | **1** |
| **Ig gamma2 chain C** | P01859 | E | **0** | **1** |
| **Guanine nucleotide-binding protein G(s), alpha subunit** | P63092 | E, Mb | **0** | **1** |
| **Carboxypeptidase B precursor** | P15086 | E, C | **0** | **1** |
| **Succinate-CoA ligase, GDP-forming, beta subunit** | Q96199 | C | **0** | **1** |
| **Inorganic pyrophosphatase 2, mitochondrial precursor** | Q9H2U2 | C | **0** | **1** |
| **Sulfide:quinone oxidoreductase, mitochondrial precursor** | Q9Y6N5 | C | **0** | **1** |
| **ADP/ATP translocase 3** | P12236 | C | **0** | **1** |
| **3-hydroxyacyl-CoA dehydrogenase type-2** | Q99714 | C, Mb | **0** | **1** |
| **Carbamoyl-phosphate synthase** | P31327 | C | **0** | **1** |
| **Glutathione S-transferase kappa 1** | Q9Y2Q3 | C | **0** | **1** |
| **Mitochondrial import receptor subunit TOM22 homolog** | Q9NS69 | C | **0** | **1** |
| **2,4-dienoyl-CoA reductase, mitochondrial precursor** | Q16698 | C | **0** | **1** |
| **3,2-trans-enoyl-CoA isomerase, mitochondrial precursor** | P42126 | C | **0** | **1** |
| **Electron transfer flavoprotein alpha-subunit** | P13804 | C | **0** | **1** |
| **ADP/ATP translocase 1** | P12235 | C | **0** | **1** |
| **Calcium-binding mitochondrial carrier protein Aralar2** | Q9UJS0 | C, Mb | **0** | **1** |
| **Prohibitin-2 (Repressor of estrogen receptor activity)** | Q99623 | C | **0** | **1** |
| **NAD-dependent malic enzyme, mitochondrial precursor** | P23368 | C | **0** | **1** |
| **Sideroflexin-1** | Q9H9B4 | C, Mb, C | **0** | **1** |
| **Adenylate kinase isoenzyme 2** | P54819 | C | **0** | **1** |
| **Splicing factor, arginine/serine-rich 1** | Q07955 | N, C | **0** | **1** |
| **Heterogeneous nuclear ribonucleoprotein L** | P14866 | N, C | **0** | **1** |
| **Proliferation-associated protein 2G4** | Q9UQ80 | N | **0** | **1** |
| **ATP-dependent RNA helicase DD13Y** | 015523 | N, C | **0** | **1** |
| **DNA repair protein RAD50** | Q92878 | N | **0** | **1** |
| **Protein SET** | Q01105 | N, C | **0** | **1** |
| **Histone H2A.z** | P17317 | N | **0** | **1** |
| **Histone H1.4** | P10412 | N | **0** | **1** |
| **Interleukin enhancer-binding factor 3** | Q12906 | N, C | **0** | **1** |
| **Non-POU domain-containing octamer-binding protein** | Q15233 | N | **0** | **1** |
| **Mitotic checkpoint protein BUB3** | 043684 | N, C | **0** | **1** |
| **Tripartite motif protein 25** | Q14258 | N, C | **0** | **1** |
| **Serine/threonine-protein kinase PRP4 homolog** | Q13523 | N | **0** | **1** |
| **Nucleosome assembly protein 1-like 4** | Q99733 | N, C | **0** | **1** |
| **Core histone macro-H2A.1** | 075367 | N | **0** | **1** |
| **Activated RNA polymerase II transcriptional coactivator p15** | P53999 | N, C | **0** | **1** |
| **Splicing factor, arginine/serine-rich 4** | Q08170 | N | **0** | **1** |
| **Lupus La protein** | P05455 | N, Mb, C | **0** | **1** |
| **High mobility group protein 2** | P26583 | N, C | **0** | **1** |
| **Chromobox protein homolog 3** | Q13185 | N | **0** | **1** |
| **Cystatin B** | P04080 | N, C | **0** | **1** |
| **High mobility group protein 4** | 015347 | N | **0** | **1** |
| **Double-stranded RNA-specific adenosine deaminase** | P55265 | N | **0** | **1** |
| **Spliceosome RNA helicase BAT1** | Q13838 | N | **0** | **1** |
| **Histone-binding protein RBBP4** | Q09028 | N | **0** | **1** |
| **ELAV-like protein 1** | Q15717 | N, C | **0** | **1** |
| **U5 small nuclear ribonucleoprotein 200kDa helicase** | 075643 | N | **0** | **1** |
| **Signal transducer and activator of transcription 3** | P40763 | N, C | **0** | **1** |
| **Far upstream element binding protein 2** | Q92945 | N, C | **0** | **1** |
| **Heterogeneous nuclear ribonucleoproteins C1/C2** | P07910 | N, C | **0** | **1** |
| **Transcription intermediary factor 1-beta** | Q13263 | N | **0** | **1** |
| **Protein NipSnap3A** | Q9UFN0 | Mb | **0** | **1** |
| **Large neutral amino acids transporter small subunit 1** | Q01650 | Mb | **0** | **1** |
| **Myristoylated alanine-rich C-kinase substrate** | P29966 | Mb, C | **0** | **1** |
| **Platelet endothelial cell adhesion molecule prec.** | P16284 | Mb | **0** | **1** |
| **Sel-1 homolog precursor** | Q9UBV2 | Mb | **0** | **1** |
| **Oligosaccharyl transferase STT3 subunit homolog** | P46977 | Mb | **0** | **1** |
| **Guanine nucleotide-binding protein G** | P04899 | Mb, C | **0** | **1** |
| **HLA class I histocompatibility antigen, B-7 α-chain precursor** | P01889 | Mb | **0** | **1** |
| **HSA class I histocompatibility antigen, Cw-5 α-chain precursor** | Q9TNN7 | Mb | **0** | **1** |
| **HLA class I histocompatibility antigen** | P13747 | Mb | **0** | **1** |
| **Gamma-glutamyltransferase 5 precursor** | P36269 | Mb | **0** | **1** |
| **Adipocyte plasma membrane-associated protein** | Q9HDC9 | Mb | **0** | **1** |
| **Flotillin-1** | 075955 | Mb | **0** | **1** |
| **Sideroflexin-3** | Q9BWM7 | Mb | **0** | **1** |
| **Defender against cell death 1** | P61803 | Mb, C | **0** | **1** |
| **Lutheran blood group glycoprotein precursor** | P50895 | Mb | **0** | **1** |
| **CD166 antigen precursor** | Q13740 | Mb, C | **0** | **1** |
| **Vesicle-associated membrane protein-associated protein A** | Q9POL0 | Mb | **0** | **1** |
| **Leukocyte surface antigen CD47 precursor** | Q08722 | Mb | **0** | **1** |
| **Complement component C9 precursor** | P02748 | Mb, E | **0** | **1** |
| **Protein KIAA0152 precursor** | Q14165 | Mb | **0** | **1** |
| **Nucleobindin-2 precursor** | P80303 | Mb, C | **0** | **1** |
| **Mucin-1 precursor** | P15941 | Mb, C, E | **0** | **1** |
| **HLA class I histocompatibility antigen** | P18462 | Mb | **0** | **1** |
| **4F2 cell-surface antigen heavy chain** | P08195 | Mb | **0** | **1** |
| **Transmembrane protein Tmp21** | P49755 | Mb | **0** | **1** |
| **Aquaporin-1** | P29972 | Mb | **0** | **1** |
| **Actin-related protein 2/3 complex subunit 1 B** | O15143 | Mb | **0** | **' 1** |
| **Integrin beta-1 precursor** | P05556 | Mb | **0** | **1** |
| **HLA class II histocompatibility antigen, DRB1-1β chain prec.** | P04229 | Mb | **0** | **1** |
| **D-3-phosphoglycerate dehydrogenase** | 043175 | U | **0** | **1** |
| **Glycogen phosphorylase, liver form** | P06737 | U | **0** | **1** |
| **Protein FAM82B** | Q96DB5 | U | **0** | **1** |
| **UDP-glucose 6-dehydrogenase** | 060701 | U | **0** | **1** |
| **SH3 domain-binding glutamic acid-rich-like protein** | 075368 | U | **0** | **1** |
| **HLA class I histocompatibility antigen, A-69 alpha chain** | P10316 | U | **0** | **1** |
| **Keratin, type I cytoskeletal 19** | P08727 | C | **1** | **10** |
| **Phosphoglycerate kinase 1** | P00558 | C, N | **1** | **9** |
| **Heat shock protein HSP 90-beta** | P08238 | C, N | **1** | **8** |
| **Actinin alpha4** | 043707 | C, N | **1** | **8** |
| **14-3-3 protein epsilon** | P62258 | C, N | **1** | **7** |
| **Synaptic vesicle membrane protein VAT-1 homolog** | Q99536 | C | **1** | **7** |
| **Heat shock 70kDa** | P08107 | C, N | **1** | **7** |
| **Protein disulfide-isomerase precursor** | P07237 | C | **1** | **7** |
| **Anterior gradient protein 2 homolog precursor** | 095994 | C | **1** | **7** |
| **Lamin Bl** | P20700 | N | **1** | **7** |
| **Cofilin-1** | P23528 | C, N, Mb | **1** | **6** |
| **ADP-ribosylation factor 1** | P84077 | C, Mb | **1** | **6** |
| **Heat-shock protein beta-1** | P04792 | C, N, Mb | **1** | **6** |
| **Ribosome-binding protein 1** | Q9P2E9 | C | **1** | **6** |
| **14-3-3 protein beta/alpha** | P31946 | C | **1** | **5** |
| **Myosin-10** | P35580 | C | **1** | **5** |
| **Elongation factor 2 EF-2** | P13639 | C | **1** | **5** |
| **Rho GDP-dissociation inhibitor 1** | P52565 | C | **1** | **5** |
| **Macrophage capping protein** | P40121 | C, N | **1** | **5** |
| **Protein disulfide-isomerase A6 precursor** | Q15084 | C | **1** | **5** |
| **Protein disulfide-isomerase A4 precursor** | P13667 | C, Mb | **1** | **5** |
| **Inter-alpha-trypsin inhibitor heavy chain H1 precursor** | P19827 | E | **1** | **5** |
| **Tenascin precursor** | P24821 | E, Mb | **1** | **5** |
| **TGF-beta induced protein IG-H3 precursor** | Q15582 | E, C, N | **1** | **5** |
| **Fibulin-1 precursor** | P23142 | E | **1** | **5** |
| **Nascent polypeptide-associated complex alpha subunit** | Q13765 | N | **1** | **5** |
| **Chloride intracellular channel protein 1** | 000299 | Mb, N | **1** | **5** |
| **Ras-related protein Rap-1A** | P62834 | Mb, N | **1** | **5** |
| **F-actin capping protein alpha-1 subunit** | P52907 | C | **1** | **4** |
| **Glutathione S-transferase P** | P09211 | C, N | **1** | **4** |
| **L-plastin** | P13796 | C | **1** | **4** |
| **Tropomyosin beta chain** | P07951 | C | **1** | **4** |
| **Stress-70 protein, mitochondrial precursor** | P38646 | C, Mb | **1** | **4** |
| **Nucleolin** | P19338 | N, C, Mb | **1** | **4** |
| **Eukaryotic initiation factor 4A-I** | P60842 | N | **1** | **4** |
| **Heterogeneous nuclear ribonucleoprotein Q** | 060506 | C, N | **1** | **3** |
| **F-actin capping protein beta subunit** | P47756 | C | **1** | **3** |
| **Actin-like protein 3** | P61158 | C | **1** | **3** |
| **14-3-3 protein eta** | Q04917 | C | **1** | **3** |
| **40S ribosomal protein S3** | P23396 | C | **1** | **3** |
| **Ras GTPase-activating-like protein** | P46940 | C, Mb | **1** | **3** |
| **Transketolase** | P29401 | C | **1** | **3** |
| **Glycogen phosphorylase brain form** | P11216 | C, N | **1** | **3** |
| **Clathrin heavy polypeptide** | Q00610 | C, Mb | **1** | **3** |
| **Tropomyosin alpha 3 chain** | P06753 | C | **1** | **3** |
| **Spectrin alpha chain, brain** | Q13813 | C, Mb | **1** | **3** |
| **Actin, alpha cardiac** | P68032 | C | **1** | **3** |
| **Ribophorin II** | P04844 | C | **1** | **3** |
| **Laminin beta-1 chain precursor** | P07942 | E | **1** | **3** |
| **Apolipoprotein A IV** | P06727 | E | **1** | **3** |
| **Apolipoprotein B-100 precursor** | P04114 | E | **1** | **3** |
| **Laminin alpha-4 chain precursor** | Q16363 | E, Mb | **1** | **3** |
| **Trifunctional enzyme alpha subunit, mitochondrial precursor** | P40939 | C | **1** | **3** |
| **Heterogenous nuclear ribonucleoprotein U** | Q00839 | N | **1** | **3** |
| **Annexin A4** | P09525 | N, C | **1** | **3** |
| **Transitional endoplasmic reticulum ATPase** | P55072 | N, C | **1** | **3** |
| **Rab GDP dissociation inhibitor beta** | P50395 | Mb, C | **1** | **3** |
| **Collagen-binding protein 2 precursor** | P50454 | Mb, C | **1** | **3** |
| **Ezrin (p81)** | P15311 | Mb | **1** | **3** |
| **Plectin 1** | Q15149 | Mb, C, N | **1** | **3** |
| **Adenine phosphoribosyltransferase** | P07741 | C | **1** | **2** |
| **ADP-ribosylation factor 4** | P18085 | C | **1** | **2** |
| **40S ribosomal protein S15a** | P62244 | C | **1** | **2** |
| **60S ribosomal protein L6** | Q02878 | C | **1** | **2** |
| **SAM domain and HD domain-containing protein 1** | Q9Y3Z3 | C | **1** | **2** |
| **Vacuolar protein sorting 26** | 075436 | C | **1** | **2** |
| **Fatty acid synthase** | P49327 | C | **1** | **2** |
| **Thioredoxin** | P10599 | C, N, E | **1** | **2** |
| **Glucosidase II beta subunit precursor** | P14314 | C | **1** | **2** |
| **Ribophorin I** | P04843 | C | **1** | **2** |
| **Ig lambda chain V-III region LOI** | P80748 | E | **1** | **2** |
| **ADP/ATP translocase 2** | P05141 | C | **1** | **2** |
| **Aconitate hydratase mitochondrial precursor** | Q99798 | C | **1** | **2** |
| **Glutamate deshydrogenase** | P00367 | C | **1** | **2** |
| **DNA-dependent protein kinase catalytic subunit** | P78527 | N | **1** | **2** |
| **Histone H1.5** | P16401 | N | **1** | **2** |
| **DEAH box protein 15** | 043143 | N | **1** | **2** |
| **Calcyclin (Prolactin receptor associated protein)** | P06703 | N, C | **1** | **2** |
| **PRA1 family protein 3** | 075915 | Mb, C | **1** | **2** |
| **Protein C7orf24** | 075223 | U | **1** | **2** |
| **Cellular retinoic acid-binding protein 1** | P29762 | C | **1** | **1** |
| **FK506-binding protein 1A** | P62942 | C | **1** | **1** |
| **60S ribosomal protein L18** | Q07020 | C | **1** | **1** |
| **Caspase-14 precursor** | P31944 | C | **1** | **1** |
| **Carbonyl reductase** | P16152 | C | **1** | **1** |
| **Copine-1** | Q99829 | C | **1** | **1** |
| **Guanine nucleotide-binding protein beta subunit 2-like 1** | P63244 | C | **1** | **1** |
| **Eukaryotic translation initiation factor 2 subunit 1** | P05198 | C | **1** | **1** |
| **Selenium-binding protein 1** | Q13228 | C, Mb | **1** | **1** |
| **4-trimethylaminobutyraldehyde dehydrogenase** | P49189 | C | **1** | **1** |
| **Glucose-6-phosphate isomerase** | P06744 | C | **1** | **1** |
| **Alcohol dehydrogenase** | P14550 | C | **1** | **1** |
| **Spectrin beta chain** | Q01082 | C, Mb | **1** | **1** |
| **Coatomer subunit gamma** | Q9Y678 | C | **1** | **1** |
| **Tubulin beta-6 chain** | Q9BUF5 | C | **1** | **1** |
| **Disheveled associated activator of morphogenesis 2** | Q86T65 | C | **1** | **1** |
| **Receptor-interacting serine/threonine-protein kinase 2** | Q13546 | C | **1** | **1** |
| **Minor histocompatibility antigen H13** | Q8TCT9 | C | **1** | **1** |
| **Sarcoplasmic/endoplasmic reticulum calcium ATPase 2** | P16615 | C | **1** | **1** |
| **Cytochrome P450 1 B1** | Q16678 | C | **1** | **1** |
| **Ras-related protein Rab-2A** | P61019 | C | **1** | **1** |
| **Plasminogen precursor** | P00747 | E | **1** | **1** |
| **Laminin beta-2 chain precursor** | P55268 | E | **1** | **1** |
| **Calgranulin B** | P06702 | E, Mb, C | **1** | **1** |
| **Ig lambda chain V-IV region Hil** | P01717 | E | **1** | **1** |
| **Protein-glutamine gamma-glutamyltransferase 2** | P21980 | E, Mb, C | **1** | **1** |
| **Collagen alpha-1(1VIII) chain precursor** | P39060 | E, C | **1** | **1** |
| **Acyl-CoA dehydrogenase, very-long-chain** | P49748 | C | **1** | **1** |
| **L3-hydroxyacyl-CoA dehydrogenase, short chain** | Q16836 | C | **1** | **1** |
| **Trifunctional enzyme beta subunit, mitochondrial precursor** | P55084 | C | **1** | **1** |
| **Voltage-dependent anion-selective channel protein 2** | P45880 | C | **1** | **1** |
| **Lethal(3)malignant brain tumor-like 3 protein** | Q96JM7 | N | **1** | **1** |
| **Histone H2A.z** | P0C0S5 | N | **1** | **1** |
| **Staphylococcal nuclease domain-containing protein 1** | Q7KZF4 | N | **1** | **1** |
| **Histone H2A type 1-A** | Q96QV6 | N | **1** | **1** |
| **Heterogeneous nuclear ribonucleoprotein R** | 043390 | N | **1** | **1** |
| **Myosin Ic** | 000159 | Mb, C | **1** | **1** |
| **CD44 antigen precursor** | P16070 | Mb | **1** | **1** |
| **Solute carrier family 2** | P11166 | Mb | **1** | **1** |
| **Vesicle trafficking protein SEC22b** | 075396 | Mb, C | **1** | **1** |
| **Protein C10orf58 precursor** | Q9BR18 | U | **1** | **1** |
| **Tryptophanyl-tRNA synthetase** | P23381 | C | **1** | **0** |
| **Glucose-6-phosphate 1-dehydrogenase** | P11413 | C | **1** | **0** |
| **Myosin regulatory light chain 2** | P24844 | C | **1** | **0** |
| **Calponin-1** | P51911 | C | **1** | **0** |
| **Keratin, type II cytoskeletal 4** | P19013 | C | **1** | **0** |
| **Perilipin** | 060240 | C | **1** | **0** |
| **Voltage-dependent L-type calcium channel alpha-1** | Q13698 | C | **1** | **0** |
| **Keratin, type I cytoskeletal 13** | P13646 | C, Mb | **1** | **0** |
| **EH-domain containing protein 2** | Q9NZN4 | C, N, Mb | **1** | **0** |
| **Dihydropyrimidinase-related protein 2** | Q16555 | C, N | **1** | **0** |
| **UTP--glucose-1-phosphate uridylyltransferase 1** | Q07131 | C | **1** | **0** |
| **Hormone-sensitive lipase** | Q05469 | C, N | **1** | **0** |
| **Aldo-keto reductase family 1 member C1** | Q04828 | C | **1** | **0** |
| **Keratin, type II cytoskeletal 1b** | Q7Z794 | C | **1** | **0** |
| **Thioredoxin domain-containing protein 5 precursor** | Q8NBS9 | C, Mb | **1** | **0** |
| **Thioredoxin domain-containing protein 1 precursor** | Q9H3N1 | C, Mb | **1** | **0** |
| **Long-chain-fatty-acid--CoA ligase 1** | P33121 | C | **1** | **0** |
| **Heparin cofactor II precursor** | P05546 | E | **1** | **0** |
| **Extracellular superoxide dismutase** | P08294 | E | **1** | **0** |
| **Nidogen-1 precursor** | P14543 | E | **1** | **0** |
| **C9orf19** | Q9H4G4 | E, Mb, C | | **0** |
| **Corticosteroid-binding globulin precursor** | P08185 | E | **1** | **0** |
| **Ig heavy chain V-III region HIL** | P01771 | E | **1** | **0** |
| **Ig kappa chain V-I region Lay** | P01605 | E | **1** | **0** |
| **Ig kappa chain V-I region OU** | P01606 | E | **1** | **0** |
| **Serum amyloid A-4 protein (candidate of metastasis 1)** | P35542 | E | **1** | **0** |
| **Galectin-3** | P17931 | E, N, Mb | **1** | **0** |
| **Tubulointerstitial nephritis antigen** | Q9GZM7 | E | **1** | **0** |
| **Heat shock protein 75 kDa, mitochondrial precursor** | Q12931 | C | **1** | **0** |
| **3-ketoacyl-CoA thiolase, mitochondrial** | P42765 | C | **1** | **0** |
| **Probable transcription factor PML** | P29590 | N | **1** | **0** |
| **Probable global transcription activator SNF2L1** | P28370 | N | **1** | **0** |
| **Ras-related protein R-Ras (p23)** | P10301 | Mb, C | **1** | **0** |
| **Target of Nesh-SH3 precursor** | Q7Z7G0 | Mb | **1** | **0** |
| **Band 3 anion transport protein** | P02730 | Mb, N, C | **1** | **0** |
| **Platelet glycoprotein 4** | P16671 | Mb | **1** | **0** |
| **Cell surface glycoprotein MUC18 precursor** | P43121 | Mb, E, C | **1** | **0** |
| **Spectrin alpha chain, erythrocyte** | P02549 | Mb | **1** | **0** |
| **Talin-2** | Q9Y4G6 | Mb | **1** | **0** |
| **Ankyrin-1** | P16157 | Mb, C | **1** | **0** |
| **Synaptotagmin-5** | 000445 | Mb | **1** | **0** |
| **Adipocyte-derived leucine aminopeptidase precursor** | Q9NZ08 | Mb, E | **1** | **0** |
| **Vacuolar ATP synthase subunit B, kidney isoform** | P15313 | Mb, C | **1** | **0** |
| **Clathrin coat assembly protein AP180** | 060641 | Mb, C | **1** | **0** |
| **78 kDa glucose-regulated protein precursor** | P11021 | C | **2** | **10** |
| **Peptidyl-prolyl cis-trans isomerase A** | P62937 | C | **2** | **9** |
| **Endoplasmin precursor 94kDa glucose-regulated protein** | P14625 | C | **2** | **9** |
| **Fructose-bisphosphate aldolase A** | P04075 | C | **2** | **8** |
| **Keratin, type II cytoskeletal 7** | P08729 | C | **2** | **8** |
| **Neuroblast differentiation associated protein AHNAK** | Q09666 | N | **2** | **8** |
| **Calgizzarin S100C** | P31949 | C, N, Mb | **2** | **7** |
| **Elongation factor 1-alpha** | P68104 | C, N | **2** | **7** |
| **Heat shock protein HSP90 -alphaHSP86** | P07900 | C, N | **2** | **7** |
| **Protein disulfide-isomerase A3 precursor** | P30101 | C, Mb | **2** | **7** |
| **Collagen alpha 1(1II)** | Q99715 | E, C | **2** | **7** |
| **60 kDa heat shock protein mitochondrial precursor** | P10809 | C, Mb | **2** | **7** |
| **Transgelin-2** | P37802 | U | **2** | **7** |
| **Heat shock 70k** | P34931 | C, N | **2** | **6** |
| **Tubulin beta 2** | P68371 | C | **2** | **6** |
| **14-3-3 protein gamma** | P61981 | C | **2** | **6** |
| **Moesin** | P26038 | C, Mb, N | **2** | **6** |
| **Peptidyl-prolyl cis-trans isomerase B precursor** | P23284 | C, E, Mb | **2** | **6** |
| **Complement factor H precursor** | P08603 | E, C | **2** | **6** |
| **Cathepsin D** | P07339 | C, E | **2** | **6** |
| **ATP synthase alpha chain** | P25705 | C | **2** | **6** |
| **Voltage-dependent anion-selective channel protein 1** | P21796 | C, Mb | **2** | **6** |
| **Histone H1.2** | P16403 | N | **2** | **5** |
| **Ras-related protein Rab-10** | P61026 | C | **2** | **4** |
| **Ubiquitin-activating enzyme E1** | P22314 | C, Mb | **2** | **4** |
| **Raf kinase inhibitor protein** | P30086 | C, Mb | **2** | **4** |
| **Complement C4-A precursor** | P0C0L4 | E, Mb | **2** | **3** |
| **Angiotensinogen precursor** | P01019 | E | **2** | **3** |
| **Coagulation factor 1III A chain precursor** | P00488 | E | **2** | **3** |
| **Tetranectin precursor** | P05452 | E | **2** | **3** |
| **Phosphate carrier protein** | Q00325 | C | **2** | **3** |
| **Thioredoxin-dependent peroxide reductase precursor** | P30048 | C | **2** | **3** |
| **Histone 1 H2AE** | P28001 | N | **2** | **3** |
| **Four and a half LIM domains protein 1 FHL** | Q13642 | C | **2** | **2** |
| **Catalase** | P04040 | C, C | **2** | **2** |
| **Ig kappa chain V-I region CAR** | P01596 | E | **2** | **2** |
| **Inter-alpha-trypsin inhibitor heavy chain H2 precursor** | P19823 | E | **2** | **2** |
| **Laminin gamma-1chain precursor** | P11047 | E, Mb | **2** | **2** |
| **Pigment epithelium-derived factor precursor** | P36955 | E | **2** | **2** |
| **Ig kappa chain V-I region AG** | P01593 | E | **2** | **2** |
| **Prolactin-inducible protein precursor** | P12273 | E | **2** | **2** |
| **Alpha-1 acid glycoprotein** | P02763 | E | **2** | **2** |
| **Ig kappa chain V-II region Cum** | P01614 | E | **2** | **2** |
| **Alpha-1B-glycoprotein precursor** | P04217 | E | **2** | **2** |
| **Basement membrane-specific HSPG core protein precursor** | P98160 | E | **2** | **2** |
| **Myosin light polypeptide 6** | P60660 | C | **2** | **1** |
| **Hemoglobin gamma-1 chain** | P69891 | C | **2** | **1** |
| **Immunoglobulin J chain** | P01591 | C, E | **2** | **1** |
| **Inter-alpha-trypsin inhibitor heavy chain H4 precursor** | Q14624 | E | **2** | **1** |
| **Complement C4 precursor** | P01028 | E | **2** | **1** |
| **Nidogen-2 precursor** | Q14112 | E | **2** | **1** |
| **Serum amyloid A protein precursor** | P02735 | E | **2** | **1** |
| **Aldehyde dehydrogenase mitochondrial precursor** | P05091 | C | **2** | **1** |
| **6-phosphogluconate dehydrogenase, decarboxylating** | P52209 | C | **2** | **0** |
| **Retinal dehydrogenase 1** | P00352 | C | **2** | **0** |
| **Transforming protein RhoA precursor** | P61586 | C, Mb, N | **2** | **0** |
| **Alcohol dehydrogenase 1A** | P07327 | C | **2** | **0** |
| **Liver carboxylesterase 1 precursor** | P23141 | C, E | **2** | **0** |
| **Kininogen-1 precursor** | P01042 | E | **2** | **0** |
| **Zinc-alpha-2-glycoprotei n precursor** | P25311 | E | **2** | **0** |
| **Alpha-2-HS-glycoprotein precursor, Fetuin** | P02765 | E | **2** | **0** |
| **Collagen alpha-2(IV) chain precursor** | P08572 | E | **2** | **0** |
| **Grainyhead-like protein 1 homolog** | Q9NZI5 | N | **2** | **0** |
| **Fibronectin precursor** | P02751 | E | **3** | **10** |
| **14-3-3 protein zeta/delta** | P63104 | C | **3** | **9** |
| **Pyruvate kinase, isozymes M1/M2** | P14618 | C | **3** | **8** |
| **Profilin-1** | P07737 | C | **3** | **7** |
| **Vinculin Metavinculin** | P18206 | C, Mb | **3** | **6** |
| **Histidine rich glycoprotein** | P04196 | E, Mb | **3** | **6** |
| **Transgelin** | Q01995 | C | **3** | **5** |
| **L-lactate dehydrogenase B chain** | P07195 | C | **3** | **5** |
| **Apolipoprotein E** | P02649 | E, C | **3** | **5** |
| **Triosephosphate isomerase 1** | P60174 | C | **3** | **4** |
| **Complement factor B precursor** | P00751 | E | **3** | **4** |
| **Lactotransferrin precursor** | P02788 | E, C, Mb | **3** | **4** |
| **Keratin, type II cytoskeletal 6A** | P02538 | C | **3** | **3** |
| **Ig kappa chain V-III region SIE** | P01620 | E | **3** | **3** |
| **Ig kappa chain V-IV region Len** | P01625 | E | **3** | **3** |
| **AMBP protein precursor** | P02760 | E, C, Mb | **3** | **3** |
| **Myosin light chain 1, slow-twitch muscle A isoform** | P14649 | C, N | **3** | **2** |
| **Apolipoprotein D precursor** | P05090 | E | **3** | **2** |
| **Ig heavy chain V-III region BRO** | P01766 | E | **3** | **2** |
| **Carbonic anhydrase 1** | P00915 | C | **3** | **1** |
| **Vitamin D binding protein** | P02774 | E, C, Mb | **3** | **1** |
| **Apolipoprotein C-III precursor** | P02656 | E | **3** | **1** |
| **Dermatopontin precursor** | Q07507 | E | **3** | **1** |
| **Collagen type I alpha 2** | P08123 | E | **3** | **1** |
| **Glycerol-3-phosphate dehydrogenase** | P21695 | C | **3** | **0** |
| **Membrane copper amine oxidase** | Q16853 | Mb | **3** | **0** |
| **Tubulin beta-2 chain** | P07437 | C | **4** | **10** |
| **Filamin A** | P21333 | C, N | **4** | **9** |
| **Peroxiredoxin 1** | Q06830 | C, N | **4** | **9** |
| **Glyceraldehyde-3-phosphatedehydrogenase** | P04406 | C, N | **4** | **9** |
| **Gelsolin precursor (Actin-depolymerizing factor)** | P06396 | E, C | **4** | **9** |
| **Heat shock cognate 71kDa protein** | P11142 | C | **4** | **8** |
| **Annexin A1** | P04083 | Mb, C, N | **4** | **8** |
| **Alpha-actinin 1** | P12814 | C | **4** | **7** |
| **Annexin A5** | P08758 | C, E, N | **4** | **7** |
| **L-lactate dehydrogenase A chain** | P00338 | C | **4** | **6** |
| **Vitronectin** | P04004 | E | **4** | **6** |
| **ATP synthase beta chain, mitochondrial precursor** | P06576 | C | **4** | **6** |
| **Immunoglobulin Am2** | P01877 | E | **4** | **5** |
| **Histone H1t** | P22492 | N, C | **4** | **4** |
| **Myosin-11** | P35749 | C, E, N | **4** | **3** |
| **Peroxiredoxin 2** | P32119 | C, Mb | **4** | **3** |
| **Keratin type II cytoskeletal 3** | P12035 | C | **4** | **3** |
| **Immunoglobin Gm 1** | P01857 | Mb | **4** | **3** |
| **Keratin, type II cytoskeletal 5** | P13647 | C | **4** | **2** |
| **Keratin, type I cytoskeletal 16** | P08779 | C | **4** | **2** |
| **Haptoglobin-related protein precursor** | P00739 | E | **4** | **2** |
| **RNA Polymerase I and transcript release factor PTRF** | Q6NZI2 | N | **4** | **2** |
| **Keratin, type I cytoskeletal 17** | Q04695 | C, N | **4** | **1** |
| **Myosin heavy polypeptide 9 non-muscle** | P35579 | C | **5** | **10** |
| **Keratin, type II cytoskeletal 8** | P05787 | C | **5** | **10** |
| **Periostin precursor** | Q15063 | E | **5** | **10** |
| **Ubiquitin** | P62988 | C | **5** | **9** |
| **Tubulin alpha** | P68363 | C | **5** | **9** |
| **Alpha enolase** | P06733 | N | **5** | **9** |
| **Antithrombin III** | P01008 | E | **5** | **6** |
| **Transthyretin precursor** | P02766 | E | **5** | **6** |
| **Plasma protease C1 inhibitor precursor** | P05155 | E | **5** | **5** |
| **Ceruloplasmin precursor** | P00450 | E | **5** | **5** |
| **Tropomyosin 1 alpha chain** | P09493 | C | **5** | **4** |
| **Asporin precursor** | Q9B1N1 | E | **5** | **4** |
| **Mast cell carboxypeptidase A precursor** | P15088 | C | **5** | **4** |
| **Fatty acid-binding protein** | P15090 | C | **5** | **2** |
| **Keratin, type I cytoskeletal 14** | P02533 | C | **5** | **1** |
| **Haptoglobin precursor (alpha and beta chains)** | P00738 | E | **6** | **9** |
| **Histone H2B a/g/h/k/l** | P62807 | N | **6** | **7** |
| **Alpha 1 antichymotrypsin** | P01011 | E, C, N | **6** | **5** |
| **Histone H4** | P62805 | N | **7** | **10** |
| **Immunoglobulin kappa light chain** | P01834 | E, N, C | **7** | **9** |
| **Clusterin precursor** | P10909 | E, N | **7** | **9** |
| **Collagen alpha 2(VI) chain precursor** | P12110 | E | **7** | **9** |
| **Fibrinogen alpha chain precursor** | P02671 | E | **7** | **7** |
| **Fibrinogen beta chain** | P02675 | E, C | **8** | **10** |
| **Annexin A2** | P07355 | N, C, E | **8** | **10** |
| **Lamin A/C** | P02545 | N, C | **8** | **10** |
| **Ig lambda chain C regions** | P01842 | E | **8** | **8** |
| **Collagen alpha1 VI chain precursor** | P12109 | E | **8** | **8** |
| **Hemoglobin delta chain** | P02042 | C | **8** | **7** |
| **Actin, aortic smooth muscle Alpha-actin-2** | P62736 | C | **8** | **6** |
| **Ig mu chain C region** | P01871 | E, Mb | **8** | **5** |
| **Serum amyloid P-component precursor** | P02743 | E, N, C | **8** | **3** |
| **Actin cytoplasmic 1 Beta** | P60709 | C | **9** | **10** |
| **Collagen alpha 1 1IV chain** | Q05707 | E | **9** | **10** |
| **Ig alpha-1chain** | P01876 | E | **9** | **10** |
| **Complement C3 precursor** | P01024 | E | **9** | **9** |
| **Prolargin precursor** | P51888 | E | **9** | **9** |
| **Fibrinogen gamma chain precursor** | P02679 | E | **9** | **8** |
| **Keratin, type I cytoskeletal 9** | P35527 | C | **9** | **7** |
| **Keratin 2E** | P35908 | C | **9** | **6** |
| **Hemoglobin alpha chain** | P69905 | C | **10** | **10** |
| **Hemoglobin beta chain** | P68871 | C | **10** | **10** |
| **Vimentin** | P08670 | C, N | **10** | **10** |
| **Collagen type VI alpha 3** | P12111 | E | **10** | **10** |
| **Lumican precursor** | P51884 | E | **10** | **10** |
| **Alpha-1-antitrypsin precursor** | P01009 | E, C | **10** | **10** |
| **Apolipoprotein A I** | P02647 | E, C | **10** | **10** |
| **Biglycan precursor (Bone/cartilage proteoglycan)** | P21810 | E, | **10** | **10** |
| **Serotransferrin precursor** | P02787 | E, C, N | **10** | **10** |
| **Decorin** | P07585 | E | **10** | **10** |
| **Serum albumin precursor** | P02768 | E | **10** | **10** |
| **Macroglobulin alpha 2** | P01023 | E | **10** | **9** |
| **Osteoglycin** | P20774 | E | **10** | **9** |
| **Keratin, type II cytoskeletal 1** | P04264 | Mb | **10** | **9** |
| **Hemopexin** | P02790 | E | **10** | **8** |
| **Keratin, type I cytoskeletal 10** | P13645 | C, N | **10** | **7** |

## Claims

1. An in-vitro method for screening specific disease biological markers which are accessible from the extra cellular space in pathologic tissues for high-affinity ligands, comprising the steps of
- immersion of a native normal tissue sample in a solution containing a labelling reagent for labelling proteins; wherein accessible proteins are labelled by the labelling reagent;
- immersion of a native pathologic tissue sample in a solution containing a labelling reagent for labelling proteins, wherein accessible proteins are labelled by the labelling reagent;
- separate purification of the labelled proteins of each of the samples;
- analysis of the labelled proteins or fragments thereof of normal tissue and pathologic tissue, respectively;
- determination of the differential expression pattern of the labelled proteins in the native pathologic tissue samples compared to the normal tissue samples;
- judging that the labelled protein(s) having higher expression in the native pathologic tissue sample compared to the normal tissue sample or being expressed more frequently in respective native pathologic tissue samples compared to the normal tissue sample is/are biological marker(s) for pathologic tissue, which are accessible for high-affinity ligands from the extra cellular space.

2. The method according to claim 1, wherein it is judged that the labelled protein(s) having expression in the native pathologic tissue sample but which is/are not or essentially not expressed in the normal tissue is/are biological marker(s) for pathologic tissue, which are accessible for high-affinity ligands from the extra cellular space, preferably are accessible for high-affinity ligands from the extra cellular space in native tissue, most preferred are accessible for high-affinity ligands from the extra cellular space *in vivo.*

3. The method according to claim 1 or 2, wherein the labelling reagent for labelling proteins is a reactive biotin, preferably a biotin reactive ester derivative.

4. The method according to any one of claims 1 to 3, wherein the purification step makes use of the label of the labelled proteins as selective marker.

5. The method according to any one of claims 1 to 4, wherein the label is a biotin residue and wherein purification is performed using streptavidin bound to a resin, wherein the biotin-labelled proteins are bound to the resin via streptavidin.

6. The method according to any one of claims 1 to 5, wherein after the purification step the labelled proteins are cleaved to peptides, preferably by proteolytic digestion.

7. The method according to any one of claims 1 to 6, wherein the analysis step comprises mass spectrometry, preferably microsequencing by tandem mass spectrometry.

8. The method according to any one of claims 1 to 7, wherein the native pathologic tissue sample is derived from tissues selected from the group consisting of tumor tissue, inflamed tissue, atheromatotic tissue or resulting from degenerative, metabolic and genetic diseases.

9. The method according to any one of claims 1 to 8, wherein accessibility of the biological markers refers to being accessible for high-affinity ligands from the extra cellular space in native tissue.

10. The method according to any one of claims 1 to 9, wherein biological markers for pathologic diseases which are not accessible for high-affinity ligands from the extracellular space in a native tissue sample will not or will essentially not be labelled.

11. The method according to claim 9 or 10, wherein the high-affinity ligands are selected from the group consisting of antibodies, antibody fragments, drugs, prodrugs, ligands, biotin, and derivatives and conjugates thereof, preferably conjugates of antibodies or antibody fragments with drugs or prodrugs.

12. The method according to any one of claims 1 to 11, wherein biological markers are proteins or polypeptides, which are expressed in the given pathologic tissue and not expressed in the normal tissue, or are expressed on a higher level in the given pathologic tissue than in the normal tissue, wherein the biological marker indicates a pathologic condition compared to the normal physiologic condition of the corresponding normal tissue.

## Patentansprüche

1. Ein *in-vitro* Verfahren zur Auswahl von für Krankheiten spezifischen biologischen Markern, die über den extrazellulären Raum pathologischer Gewebe für hochaffine Liganden zugänglich sind, umfassend die folgenden Schritte:
- Eintauchen einer nativen normalen Gewebeprobe in eine Lösung enthaltend ein Markierungsreagens zum Markieren von Proteinen, wobei zugängliche Proteine durch das Markierungsreagens markiert werden;
- Eintauchen einer nativen pathologischen Gewebeprobe in eine Lösung enthaltend ein Markierungsreagens zum Markieren von Proteinen, wobei zugängliche Proteine durch das Markierungsreagens markiert werden;
- getrennte Aufreinigung der markierten Proteine von jeder der Proben;
- Analyse der markierten Proteine oder deren Fragmente aus normalem Gewebe beziehungsweise aus pathologischem Gewebe;
- Bestimmung der Unterschiede im Expressionsmuster der markierten Proteine aus der nativen pathologischen Gewebeprobe verglichen mit der normalen Gewebeprobe;
- Beurteilen, dass das oder die markierten Protein(e), die eine höhere Expression in der nativen pathologischen Gewebeprobe verglichen mit der normalen Gewebeprobe aufweisen, oder dass diese häufiger in den entsprechenden nativen pathologischen Gewebeproben verglichen mit den normalen Gewebeproben exprimiert werden, biologische Marker für pathologische Gewebe darstellen, die für hochaffine Liganden über den extrazellulären Raum pathologischer Gewebe zugänglich sind.

2. Das Verfahren gemäß Anspruch 1, wobei beurteilt wird, dass das oder die markierten Protein(e), die eine höhere Expression in der nativen pathologischen Gewebeprobe aufweisen, aber das oder die in der normalen Gewebeprobe nicht oder im wesentlichen nicht exprimiert werden, biologische Marker für pathologische Gewebe darstellen, die für hochaffine Liganden über den extrazellulären Raum pathologischer Gewebe zugänglich sind, vorzugsweise für hochaffine Liganden über den extrazellulären Raum im nativen Gewebe, am meisten bevorzugt für hochaffine Liganden *in vivo* über den extrazellulären Raum zugänglich sind.

3. Das Verfahren gemäß Anspruch 1 oder 2, wobei das Markierungsreagens für Proteine ein reaktives Biotin darstellt, vorzugsweise ein reaktives Biotinesterderivat darstellt.

4. Das Verfahren gemäß irgend einem der Ansprüche 1 bis 3, wobei im Aufreinigungsschritt von der Markierung der markierten Proteine als Selektionsmarker Gebrauch gemacht wird.

5. Das Verfahren gemäß irgend einem der Ansprüche 1 bis 4, wobei die Markierung von einem Biotinrest dargestellt wird und wobei die Aufreinigung unter Verwendung von an ein Harz gebundenes Streptavidin durchgeführt wird, wobei die mit Biotin markierten Proteine über das Streptavidin an das Harz gebunden werden.

6. Das Verfahren gemäß irgend einem der Ansprüche 1 bis 5, wobei nach dem Aufreinigungsschritt die markierten Proteine durch Spaltung in Peptide überführt werden, vorzugsweise durch proteolytische Spaltung.

7. Das Verfahren gemäß irgend einem der Ansprüche 1 bis 6, wobei der Analyseschritt Massenspektrometrie, vorzugsweise Mikrosequenzierung durch Tandem-Massenspektrometrie beinhaltet.

8. Das Verfahren gemäß irgend einem der Ansprüche 1 bis 7, wobei die native pathologische Gewebeprobe aus Geweben stammt ausgewählt aus der Gruppe bestehend aus Tumorgewebe, entzündetes Gewebe, atheromatöses Gewebe oder aus Geweben stammt, die von degenerativen Krankheiten, Stoffwechselkrankheiten oder Erbkrankheiten befallen sind.

9. Das Verfahren gemäß irgend einem der Ansprüche 1 bis 8, wobei die Zugänglichkeit der biologischen Marker sich auf die Zugänglichkeit für hochaffine Liganden über den extrazellulären Raum nativer Gewebe bezieht.

10. Das Verfahren gemäß irgend einem der Ansprüche 1 bis 9, wobei die biologischen Marker für pathologische Krankheiten, die für hochaffine Liganden über den extrazellulären Raum in einer nativen Gewebeprobe nicht zugänglich sind, nicht oder im wesentlichen nicht markiert werden.

11. Das Verfahren gemäß Anspruch 9 oder 10, wobei die hochaffinen Liganden ausgewählt sind aus der Gruppe bestehend aus Antikörpern, Antikörperfragmenten, Arzneimittelwirkstoffen, Prodrugs, Liganden, Biotin, und Derivate und Konjugate davon, vorzugsweise Konjugate von Antikörpern oder Antikörperfragmenten mit Arzneimittelwirkstoffen oder Prodrugs.

12. Das Verfahren gemäß irgend einem der Ansprüche 1 bis 11, wobei die biologischen Marker Proteine oder Polypeptide darstellen, die in einem gegebenen pathologischen Gewebe exprimiert werden und in einem normalen Gewebe nicht exprimiert werden, oder in einem gegebenen pathologischen Gewebe höher exprimiert werden als in dem normalen Gewebe, wobei der biologische Marker einen pathologischen Zustand im Vergleich mit dem normalen physiologischen Zustand eines entsprechenden normalen Gewebes anzeigt.

## Revendications

1. Procédé de détection *in vitro* de marqueurs biologiques spécifiques d'une maladie, qui sont accessibles via l'espace extracellulaire dans des tissus pathologiques aux ligands de haute affinité, comprenant les étapes consistant à :
- immerger un échantillon tissulaire normal natif dans une solution contenant un réactif de marquage pour marquer les protéines ; les protéines accessibles étant marquées par le réactif de marquage ;
- immerger un échantillon tissulaire pathologique natif dans une solution contenant un réactif de marquage pour marquer les protéines, les protéines accessibles étant marquées par le réactif de marquage ;
- séparer la purification des protéines marquées de chacun des échantillons ;
- analyser les protéines marquées ou des fragments de celles-ci de tissu normal et de tissu pathologique, respectivement ;
- déterminer le schéma d'expression différentiel des protéines marquées dans les échantillons de tissu pathologique natifs par rapport aux échantillons de tissu normal ;
- évaluer que la/les protéine(s) marquée(s) ayant une expression supérieure dans l'échantillon de tissu pathologique natif par rapport à l'échantillon tissulaire normal ou étant exprimée(s) plus fréquemment dans des échantillons de tissu pathologique natif respectif par rapport à l'échantillon de tissu normal est/sont un/des marqueur(s) biologique(s) du tissu pathologique, qui sont accessibles aux ligands de haute affinité via l'espace extracellulaire.

2. Procédé selon la revendication 1, dans lequel on détermine que la/les protéine(s) marquée(s) ayant une expression dans l'échantillon de tissu pathologique natif mais qui n'est pas/ne sont pas ou essentiellement pas exprimée(s) dans le tissu normal est/sont un/des marqueurs biologique(s) du tissu pathologique qui sont accessibles aux ligands de haute affinité via l'espace extracellulaire, de manière préférée entre toutes, sont accessibles aux ligands de haute affinité via l'espace extracellulaire *in vivo.*

3. Procédé selon la revendication 1 ou 2, dans lequel le réactif de marquage pour le marquage des protéines est une biotine réactive, de préférence, un dérivé ester réactif de la biotine.

4. Procédé selon l'une quelconque des revendications 1 à 3, dans lequel l'étape de purification utilise le marquage des protéines marquées comme marqueur sélectif.

5. Procédé selon l'une quelconque des revendications 1 à 4, dans lequel le marqueur est un résidu de biotine et dans lequel la purification est réalisée à l'aide de streptavidine liée à une résine, les protéines marquées avec la biotine étant liées à la résine via la streptavidine.

6. Procédé selon l'une quelconque des revendications 1 à 5, dans lequel, après l'étape de purification, les protéines marquées sont clivées en peptides, de préférence par digestion protéolytique.

7. Procédé selon l'une quelconque des revendications 1 à 6, dans lequel l'étape d'analyse comprend la spectrométrie de masse, de préférence, le microséquençage par spectrométrie de masse en tandem.

8. Procédé selon l'une quelconque des revendications 1 à 7, dans lequel l'échantillon de tissu pathologique est dérivé de tissus choisis dans le groupe comprenant un tissu tumoral, un tissu enflammé, un tissu athéromateux ou résultant de maladies dégénératives, métaboliques et génétiques.

9. Procédé selon l'une quelconque des revendications 1 à 8, dans lequel l'accessibilité des marqueurs biologiques fait référence au fait qu'ils sont accessibles à des ligands de haute affinité via l'espace extracellulaire dans le tissu natif.

10. Procédé selon l'une quelconque des revendications 1 à 9, dans lequel les marqueurs biologiques de maladies pathologiques qui ne sont pas accessibles aux ligands de haute affinité via l'espace extracellulaire dans un échantillon de tissu natif ne seront pas marqués ou essentiellement pas marqués.

11. Procédé selon la revendication 9 ou 10, dans lequel les ligands de haute affinité sont choisis dans le groupe comprenant les anticorps, les fragments d'anticorps, les promédicaments, les ligands, la biotine et les dérivés et conjugués de ceux-ci, de préférence, des conjugués d'anticorps ou des fragments d'anticorps avec des médicaments ou des promédicaments.

12. Procédé selon l'une quelconque des revendications 1 à 11, dans lequel les marqueurs biologiques sont des protéines ou des polypeptides qui sont exprimés dans le tissu pathologique donné et ne sont pas exprimés dans le tissu normal ou sont exprimés à un niveau supérieur dans le tissu pathologique donné par rapport à l'expression dans le tissu normal, le marqueur biologique indiquant un état pathologique par rapport à l'état physiologique normal du tissu normal correspondant.
